# EUROPEAN PATENT SPECIFICATION

(11) **EP 1 830 850 B1**
(45) Date of publication and mention of the grant of the patent: **16.03.2011**
(21) Application number: 05815816.3
(22) Date of filing: 08.12.2005
(51) Int. Cl.: A61K 31/47, A61K 31/4704, A61P 31/06

(54) **QUINOLINE DERIVATIVES FOR THE TREATMENT OF LATENT TUBERCULOSIS**
CHINOLIN-DERIVATE ZUR BEHANDLUNG VON LATENTER TUBERKULOSE
DERIVES DE QUINOLINE DESTINES AU TRAITEMENT DE LA TUBERCULOSE LATENTE

(30) Priority: 24.12.2004 EP 04078529; 08.06.2005 EP 05105008
(43) Date of publication of application: 12.09.2007
(73) Proprietor: Janssen Pharmaceutica NV, 2340 Beerse (BE)
(72) Inventor: ANDRIES, Koenraad Jozef Lodewijk Marcel, 2340 Beerse (BE); KOUL, Anil, 2340 Beerse (BE)
(74) Representative: Vervoort, Liesbeth
(86) International application number: PCT/EP2005/056594
(87) International publication number: WO 2006/067048

(56) References cited:
- WO-A-2004/011436
- NUERMBERGER ERIC ET AL: "Latent tuberculosis infection." SEMINARS IN RESPIRATORY AND CRITICAL CARE MEDICINE. JUN 2004, vol. 25, no. 3, June 2004 (2004-06), pages 317-336, XP009061348 ISSN: 1069-3424
- ANDRIES K ET AL: "A DIARYLQUINOLINE DRUG ACTIVE ON THE ATP SYNTHASE OF MYCOBACTERIUM TUBERCULOSIS" SCIENCE, AMERICAN ASSOCIATION FOR THE ADVANCEMENT OF SCIENCE,, US, vol. 307, 14 January 2005 (2005-01-14), pages 223-227, XP002358962 ISSN: 0036-8075

## Description

The present invention relates to the compound of formula (Ia) or (Ib) for use in the treatment of latent tuberculosis.

### BACKGROUND OF THE INVENTION

*Mycobacterium tuberculosis* results in more than 2 million deaths per year and is the leading cause of mortality in people infected with HIV¹. In spite of decades of tuberculosis (TB) control programs, about 2 billion people are infected by *M. tuberculosis,* though asymptomatically. About 10% of these individuals are at risk of developing active TB during their lifespan². The global epidemic of TB is fuelled by infection of HIV patients with TB and rise of multi-drug resistant TB strains (MDR-TB). The reactivation of latent TB is a high risk factor for disease development and accounts for 32% deaths in HIV infected individuals¹. To control TB epidemic, the need is to discover new drugs that can kill dormant or latent TB bacilli. The dormant TB can get reactivated to cause disease by several factors like suppression of host immunity by use of immunosuppressive agents like antibodies against tumor necrosis factor α or interferon-γ. In case of HIV positive patients the only prophylactic treatment available for latent TB is two- three months regimens of rifampicin, pyrazinamide^{3,4}. The efficacy of the treatment regime is still not clear and furthermore the length of the treatments is an important constrain in resource-limited environments. Hence there is a drastic need to identify new drugs, which can act as chemoprophylatic agents for individuals harboring latent TB bacilli.

The tubercle bacilli enter healthy individuals by inhalation; they are phagocytosed by the alveolar macrophages of the lungs. This leads to potent immune response and formation of granulomas, which consist of macrophages infected with *M. tuberculosis* surrounded by T cells. After a period of 6-8 weeks the host immune response cause death of infected cells by necrosis and accumulation of caseous material with certain extracellular bacilli, surrounded by macrophages, epitheloid cells and layers of lymphoid tissue at the periphery⁵. In case of healthy individuals, most of the mycobacteria are killed in these environments but a small proportion of bacilli still survive and are thought to exist in a non-replicating, hypometabolic state and are tolerant to killing by anti-TB drugs like isoniazid⁶. These bacilli can remain in the altered physiological environments even for individual's lifetime without showing any clinical symptoms of disease. However, in 10% of the cases these latent bacilli may reactivate to cause disease. One of the hypothesis about development of these persistent bacteria is patho-physiological environment in human lesions namely, reduced oxygen tension, nutrient limitation, and acidic pH⁷. These factors have been postulated to render these bacteria phenotypically tolerant to major anti-mycobacterial drugs⁷.

WO 2004/011436 describes substituted quinoline derivatives useful for the treatment of mycobacterial diseases. Said document discloses the antimycobacterial property of the substituted quinoline derivatives against sensitive, susceptible Mycobacterium strains but is silent on their activity against latent, dormant, persistent mycobacteria.

We have now found that the compounds of WO 2004/011436, in particular the compounds of formula (Ia) and (Ib) as defined hereinbelow, have sterilizing properties; are effective in killing dormant, latent, persistent mycobacteria, in particular *Mycobacterium tuberculosis,* and can consequently be used to treat latent TB. They will therefore greatly enhance the arsenal to fight TB.

### DESCRIPTION OF THE FIGURES

Figure 1: The effect of various drugs on dormant *M. bovis* assayed by Luciferase counts (RLU : relative luminescence units) (the bacteria were suspended in drug free medium for 5 days after 7 days of anaerobiosis).
Figure 2A): The effect of various drugs on dormant *M. bovis* (CFU: colony forming units) (CFU determined 2 days after anaerobiosis, are reported).
Figure 2B): The effect of various drugs on dormant *M. bovis* (CFU: colony forming units) (CFU determined 5 days after anaerobiosis, are reported).
Figure 3 : The effect of various drugs on dormant *M*. *tuberculosis* (Wayne model)

### INVENTION

Thus, the present invention relates to the use of a compound of formula (Ia) or (Ib) for the manufacture of a medicament for the treatment of latent tuberculosis, wherein the compound of formula (Ia) or (Ib) is 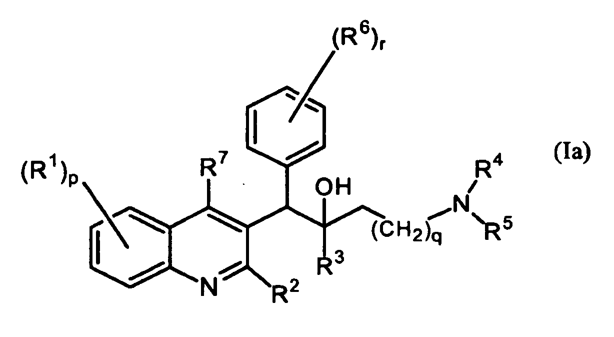 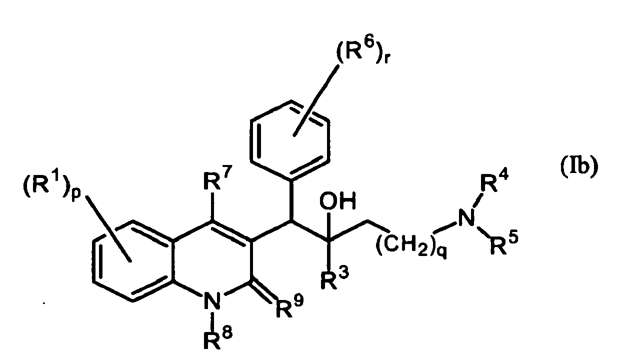 a pharmaceutically acceptable acid or base addition salt thereof, a quaternary amine thereof, a *N-*oxide thereof, a tautomeric form thereof or a stereochemically isomeric form thereof wherein
- R¹: is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
- p: is an integer equal to 1, 2, 3 or 4 ;
- R²: is hydrogen, hydroxy, mercapto, alkyloxy, alkyloxyalkyloxy, alkylthio, mono or di(alkyl)amino or a radical of formula 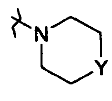 wherein Y is CH₂, O, S, NH or *N*-alkyl;
- R³: is alkyl, Ar, Ar-alkyl, Het or Het-alkyl;
- q: is an integer equal to zero, 1, 2, 3 or 4 ;
- R⁴ and R⁵: each independently are hydrogen, alkyl or benzyl; or
- R⁴ and R⁵: together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl,
- R⁶: pyridazinyl, pyrimidinyl, pyrazinyl, triazinyl, morpholinyl and thiomorpholinyl, each of said ring systems optionally substituted with alkyl, halo, haloalkyl, hydroxy, alkyloxy, amino, mono- or dialkylamino, alkylthio, alkyloxyalkyl, alkylthioalkyl and pyrimidinyl ; is hydrogen, halo, haloalkyl, hydroxy, Ar, alkyl, alkyloxy, alkylthio, alkyloxyalkyl, alkylthioalkyl, Ar-alkyl or di(Ar)alkyl ; or
- two vicinal R⁶: radicals may be taken together to form a bivalent radical of formula -CH=CH-CH=CH-;
- r: is an integer equal to 1, 2, 3, 4 or 5;
- R⁷: is hydrogen, alkyl, Ar or Het;
- R⁸: is hydrogen or alkyl;
- R⁹: is oxo ; or
- R⁸ and R⁹: together form the radical =N-CH=CH-;
- alkyl: is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo, hydroxy, alkyloxy or oxo ;
- Ar: is a homocycle selected from the group of phenyl, naphthyl, acenaphthyl, tetrahydronaphthyl, each homocycle optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
- Het: is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3- dihydrobenzo[1,4]dioxinyl or benzo[1,3]dioxolyl; each monocyclic and bicyclic heterocycle may optionally be substituted with 1,2 or 3 substituents selected from the group of halo, hydroxy, alkyl, alkyloxy, or Ar-carbonyl;
- halo: is a substituent selected from the group of fluoro, chloro, bromo and iodo; and
- haloalkyl: is a straight or branched saturated hydrocarbon radical having from 1 to 6 carbon atoms or a cyclic saturated hydrocarbon radical having from 3 to 6 carbon atoms, wherein one or more carbon atoms are substituted with one or more halo-atoms.

The compounds according to formula (Ia) and (Ib) are interrelated in that e.g. a compound according to formula (Ib), with R⁹ equal to oxo is the tautomeric equivalent of a compound according to formula (Ia) with R² equal to hydroxy (keto-enol tautomerism).

In the framework of this application, alkyl is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo, hydroxy, alkyloxy or oxo. Preferably, alkyl is methyl, ethyl or cyclohexylmethyl.

In the framework of this application, Ar is a homocycle selected from the group of phenyl, naphthyl, acenaphthyl, tetrahydronaphthyl, each optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl. Preferably, Ar is naphthyl or phenyl, each optionally substituted with 1 or 2 halo substituents.

In the framework of this application, Het is a monocyclic heterocycle selected from the group of *N-*phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle may optionally be substituted with 1, 2 or 3 substituents selected from the group of halo, hydroxy, alkyl, alkyloxy or Ar-carbonyl. Preferably, Het is thienyl.

In the framework of this application, halo is a substituent selected from the group of fluoro, chloro, bromo and iodo and haloalkyl is a straight or branched saturated hydrocarbon radical having from 1 to 6 carbon atoms or a cyclic saturated hydrocarbon radical having from 3 to 6 carbon atoms, wherein one or more carbon atoms are substituted with one or more halo-atoms. Preferably, halo is bromo, fluoro or chloro and preferably, haloalkyl is polyhaloC₁₋₆alkyl which is defined as mono- or polyhalosubstituted C₁₋₆alkyl, for example, methyl with one or more fluoro atoms, for example, difluoromethyl or trifluoromethyl, 1,1-difluoro-ethyl and the like. In case more than one halogen atoms are attached to an alkyl group within the definition of polyhaloC₁₋₆alkyl, they may be the same or different. C₁₋₆alkyl is a straight or branched saturated hydrocarbon radical having from 1 to 6 carbon atoms such as for example methyl, ethyl, propyl, 2-methyl-ethyl, pentyl, hexyl and the like.

In the definition of Het, or when R⁴ and R⁵ are taken together, it is meant to include all the possible isomeric forms of the heterocycles, for instance, pyrrolyl comprises 1*H*-pyrrolyl and 2*H*-pyrrolyl.

The Ar or Het listed in the definitions of the substituents of the compounds of formula (Ia) or (Ib) (see for instance R³) as mentioned hereinbefore or hereinafter may be attached to the remainder of the molecule of formula (Ia) or (Ib) through any ring carbon or heteroatom as appropriate, if not otherwise specified. Thus, for example, when Het is imidazolyl, it may be 1-imidazolyl, 2-imidazolyl, 4-imidazolyl and the like.

Lines drawn from substituents into ring systems indicate that the bond may be attached to any of the suitable ring atoms.

When two vicinal R⁶ radicals are taken together to form a bivalent radical of formula -CH=CH-CH=CH-, this means that the two vicinal R⁶ radicals form together with the phenyl ring to which they are attached a naphthyl.

For therapeutic use, salts of the compounds of formula (Ia) or (Ib) are those wherein the counterion is pharmaceutically acceptable. However, salts of acids and bases which are non-pharmaceutically acceptable may also find use, for example, in the preparation or purification of a pharmaceutically acceptable compound All salts, whether pharmaceutically acceptable or not, are included within the ambit of the present invention.

The pharmaceutically acceptable addition salts as mentioned hereinabove or hereinafter are meant to comprise the therapeutically active non-toxic acid addition salt forms which the compounds of formula (Ia) or (Ib) are able to form. The latter can conveniently be obtained by treating the base form with such appropriate acids as inorganic acids, for example, hydrohalic acids, e.g. hydrochloric, hydrobromic and the like; sulfuric acid; nitric acid; phosphoric acid and the like; or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely the salt form can be converted by treatment with alkali into the free base form.

The compounds of formula (Ia) or (Ib) containing acidic protons may be converted into their therapeutically active non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline, the benzathine, *N*-methyl-D-glucamine, 2-amino-2-(hydroxymethyl)-1,3-propanediol, hydrabamine salts, and salts with amino acids such as, for example, arginine, lysine and the like. Conversely the salt form can be converted by treatment with acid into the free acid form.

The term addition salt also comprises the hydrates and solvent addition forms which the compounds of formula (Ia) or (Ib) are able to form. Examples of such forms are e.g. hydrates, alcoholates and the like.

The term "quaternary amine" as used hereinbefore defines the quaternary ammonium salts which the compounds of formula (Ia) or (Ib) are able to form by reaction between a basic nitrogen of a compound of formula (Ia) or (Ib) and an appropriate quaternizing agent, such as, for example, an optionally substituted alkylhalide, arylhalide, alkylcarbonylhalide, arylcarbonylhalide, or arylalkylhalide, e.g. methyliodide or benzyliodide. Other reactants with good leaving groups may also be used, such as alkyl trifluoromethanesulfonates, alkyl methanesulfonates, and alkyl *p*-toluenesulfonates. A quaternary amine has a positively charged nitrogen. Pharmaceutically acceptable counterions include chloro, bromo, iodo, trifluoroacetate, acetate, triflate, sulfate, sulfonate. The counterion of choice can be introduced using ion exchange resins.

Compounds of either formula (Ia) and (Ib) and some of the intermediate compounds invariably have at least two stereogenic centers in their structure which may lead to at least 4 stereochemically different structures.

The term "stereochemically isomeric forms" as used hereinbefore or hereinafter defines all the possible stereoisomeric forms which the compounds of formula (Ia) and (Ib), and their quaternary amines, *N*-oxides, addition salts or physiologically functional derivatives may possess. Unless otherwise mentioned or indicated, the chemical designation of compounds denotes the mixture of all possible stereochemically isomeric forms, said mixtures containing all diastereomers and enantiomers of the basic molecular structure.

In particular, stereogenic centers may have the R- or S-configuration; substituents on bivalent cyclic (partially) saturated radicals may have either the *cis-* or *trans-* configuration. Compounds encompassing double bonds can have an E (entgegen) or Z (zusammen) -stereochemistry at said double bond. The terms cis, trans, R, S, E and Z are well known to a person skilled in the art.

Stereochemically isomeric forms of the compounds of formula (Ia) and (Ib) are obviously intended to be embraced within the scope of this invention.

Following CAS-nomenclature conventions, when two stereogenic centers of known absolute configuration are present in a molecule, an *R* or *S* descriptor is assigned (based on Cahn-Ingold-Prelog sequence rule) to the lowest-numbered chiral center, the reference center. The configuration of the second stereogenic center is indicated using relative descriptors [*R**,*R**] or [*R**,*S**], where *R** is always specified as the reference center and [*R**,*R**] indicates centers with the same chirality and [*R**,*S**] indicates centers of unlike chirality. For example, if the lowest-numbered chiral center in the molecule has an *S* configuration and the second center is *R*, the stereo descriptor would be specified as *S*-[*R**,*S**]. If "*α*" and "*β*" are used : the position of the highest priority substituent on the asymmetric carbon atom in the ring system having the lowest ring number, is arbitrarily always in the "*α*" position of the mean plane determined by the ring system. The position of the highest priority substituent on the other asymmetric carbon atom in the ring system relative to the position of the highest priority substituent on the reference atom is denominated "*α*", if it is on the same side of the mean plane determined by the ring system, or "*β*", if it is on the other side of the mean plane determined by the ring system.

When a specific stereoisomeric form is indicated, this means that said form is substantially free, i.e. associated with less than 50 %, preferably less than 20 %, more preferably less than 10%, even more preferably less than 5%, further preferably less than 2% and most preferably less than 1% of the other isomer(s). Thus, when a compound of formula (I) is for instance specified as (αS, (βR), this means that the compound is substantially free of the (αR, β S) isomer.

The compounds of either formula (Ia) and (Ib) may be synthesized in the form of racemic mixtures of enantiomers which can be separated from one another following art-known resolution procedures. The racemic compounds of either formula (Ia) and (Ib) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of either formula (Ia) and (Ib) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The tautomeric forms of the compounds of either formula (Ia) and (Ib) are meant to comprise those compounds of either formula (Ia) and (Ib) wherein e.g. an enol group is converted into a keto group (keto-enol tautomerism).

The *N-*oxide forms of the compounds according to either formula (Ia) and (Ib) are meant to comprise those compounds of either formula (Ia) and (Ib) wherein one or several tertiary nitrogen atoms are oxidized to the so-called *N*-oxide.

The compounds of formula (Ia) and (Ib) may be converted to the corresponding *N-*oxide forms following art-known procedures for converting a trivalent nitrogen into its *N-*oxide form. Said *N-*oxidation reaction may generally be carried out by reacting the starting material of formula (Ia) and (Ib) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. t.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

The invention also comprises derivative compounds (usually called "pro-drugs") of the pharmacologically-active compounds according to the invention, which are degraded *in vivo* to yield the compounds according to the invention. Pro-drugs are usually (but not always) of lower potency at the target receptor than the compounds to which they are degraded. Pro-drugs are particularly useful when the desired compound has chemical or physical properties that make its administration difficult or inefficient For example, the desired compound may be only poorly soluble, it may be poorly transported across the mucosal epithelium, or it may have an undesirably short plasma half-life. Further discussion on pro-drugs may be found in Stella, V. J. et al., "Prodrugs", Drug Delivery Systems, 1985, pp. 112-176, and Drugs, 1985, 29, pp. 455-473.

Pro-drug forms of the pharmacologically-active compounds according to the invention will generally be compounds according to either formula (Ia) and (Ib), the pharmaceutically acceptable acid or base addition salts thereof, the quaternary amines thereof, the stereochemically isomeric forms thereof, the tautomeric forms thereof and the *N-*oxide forms thereof, having an acid group which is esterified or amidated. Included in such esterified acid groups are groups of the formula -COOR^{x}, where R^{x} is a C₁₋₆alkyl, phenyl, benzyl or one of the following groups:

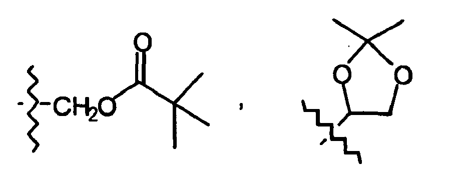

Amidated groups include groups of the formula - CONR^{y}R^{z}, wherein R^{y} is H, C₁₋₆alkyl, phenyl or benzyl and R^{z} is -OH, H, C₁₋₆alkyl, phenyl or benzyl.

Compounds according to the invention having an amino group may be derivatised with a ketone or an aldehyde such as formaldehyde to form a Mannich base. This base will hydrolyze with first order kinetics in aqueous solution.

Whenever used herein, the term "compounds of formula (Ia) or (Ib)" is meant to also include their pharmaceutically acceptable acid or base addition salts, their quaternary amines, their *N-*oxide forms, their tautomeric forms or their stereochemically isomeric forms. Of special interest are those compounds of formula (Ia) or (Ib) which are stereochemically pure.

A first interesting embodiment of the present invention relates to the use as defined hereinbefore of compounds of formula (Ia) or (Ib), wherein the compound of formula (Ia) or (Ib) is 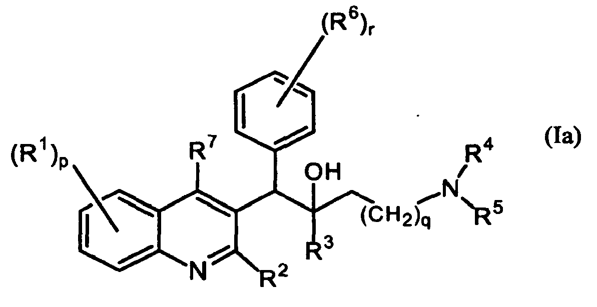 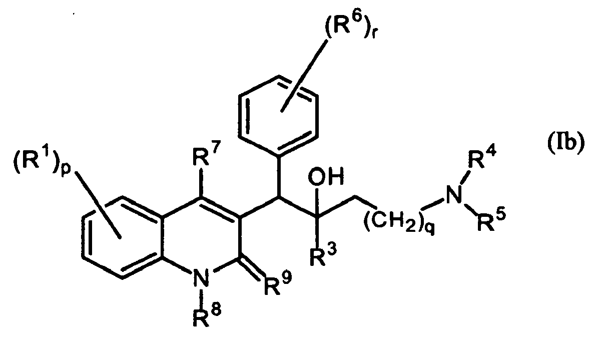 a pharmaceutically acceptable acid or base addition salt thereof, a *N-*oxide thereof, a tautomeric form thereof or a stereochemically isomeric form thereof wherein
- R¹: is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
- p: is an integer equal to 1, 2, 3 or 4 ;
- R²: is hydrogen, hydroxy, mercapto, alkyloxy, alkyloxyalkyloxy, alkylthio, mono or di(alkyl)amino or a radical of formula 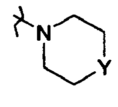 wherein Y is CH₂, O, S, NH or *N*-alkyl;
- R³: is alkyl, Ar, Ar-alkyl, Het or Het-alkyl;
- q: is an integer equal to zero, 1, 2, 3 or 4 ;
- R⁴ and R⁵: each independently are hydrogen, alkyl or benzyl; or
- R⁴ and R⁵: together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, pyridazinyl, pyrimidinyl, pyrazinyl, triazinyl, morpholinyl and thiomorpholinyl, each of said ring systems optionally substituted with alkyl halo, haloalkyl, hydroxy, alkyloxy, amino, mono- or dialkylamino, alkylthio, alkyloxyalkyl, alkylthioalkyl and pyrimidinyl ;
- R⁶: is hydrogen, halo, haloalkyl, hydroxy, Ar, alkyl, alkyloxy, alkylthio, alkyloxyalkyl alkylthioalkyl, Ar-alkyl or di(Ar)alkyl ; or
- two vicinal R⁶: radicals may be taken together to form a bivalent radical of formula -CH=CH-CH=CH-;
- r: is an integer equal to 1,2, 3, 4 or 5;
- R⁷: is hydrogen, alkyl, Ar or Het ;

- R⁸: is hydrogen or alkyl;
- R⁹: is oxo ; or
- R⁸ and R⁹: together form the radical =N-CH=CH-;
- alkyl: is a straight or branched saturated hydrocarbon radical having from 1 to 6 carbon atoms; or is a cyclic saturated hydrocarbon radical having from 3 to 6 carbon atoms; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo, hydroxy, alkyloxy or oxo ;
- Ar: is a homocycle selected from the group of phenyl, naphthyl, acenaphthyl, tetrahydronaphthyl, each homocycle optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
- Het: is a monocyclic heterocycle selected from the group of *N-*phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3- dihydrobenzo[1,4]dioxinyl or benzo[1,3]dioxolyl; each monocyclic and bicyclic heterocycle may optionally be substituted with 1, 2 or 3 substituents selected from the group of halo, hydroxy, alkyl, alkyloxy, or Ar-carbonyl;
- halo: is a substituent selected from the group of fluoro, chloro, bromo and iodo; and
- haloalkyl: is a straight or branched saturated hydrocarbon radical having from 1 to 6 carbon atoms or a cyclic saturated hydrocarbon radical having from 3 to 6 carbon atoms, wherein one or more carbon atoms are substituted with one or more halo-atoms.

A second interesting embodiment of the present invention relates to the use as defined hereinbefore of compounds of formula (Ia) or (Ib) or any subgroup thereof as mentioned hereinbefore as interesting embodiment wherein
- R¹: is hydrogen, halo, cyano, Ar, Het, alkyl, and alkyloxy ;
- p: is an integer equal to 1, 2, 3 or 4 ; in particular 1 or 2;
- R²: is hydrogen, hydroxy, alkyloxy, alkyloxyalkyloxy, alkylthio or a radical of
formula 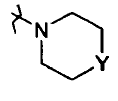 wherein Y is O;
- R³: is alkyl, Ar, Ar-alkyl or Het ;
- q: is an integer equal to zero, 1, 2, or 3;
- R⁴ and R⁵: each independently are hydrogen, alkyl or benzyl; or
- R⁴ and R⁵: ogether and including the N to which they are attached may form a radical selected from the group of pyrrolidinyl, imidazolyl, triazolyl, piperidinyl, piperazinyl, pyrazinyl, morpholinyl and thiomorpholinyl, each ring system optionally substituted with alkyl or pyrimidinyl ;
- R⁶: is hydrogen, halo or alkyl; or
- two: vicinal R⁶ radicals may be taken together to form a bivalent radical of formula -CH=CH-CH=CH-;
- r: is an integer equal to 1;
- R⁷: is hydrogen ;
- R⁸: is hydrogen or alkyl;
- R⁹: is oxo; or
- R⁸ and R⁹: together form the radical =N-CH=CH-;
- alkyl: is a straight or branched saturated hydrocarbon radical having from 1 to 6 carbon atoms ; or is a cyclic saturated hydrocarbon radical having from 3 to 6 carbon atoms ; or is a a cyclic saturated hydrocarbon radical having from 3 to 6 carbon atoms attached to a straight or branched saturated hydrocarbon radical having from 1 to 6 carbon atoms; wherein each carbon atom can be optionally substituted with halo or hydroxy;
- Ar: is a homocycle selected from the group of phenyl, naphthyl, acenaphthyl, tetrahydronaphthyl, each homocycle optionally substituted with 1, 2 or 3 substituents, each substituent independently selected from the group of halo, haloalkyl, cyano, alkyloxy and morpholinyl ;
- Het: is a monocyclic heterocycle selected from the group of *N-*phenoxypiperidinyl, piperidinyl, furanyl, thienyl, pyridinyl, pyrimidinyl ; or a bicyclic heterocycle selected from the group of benzothienyl, 2,3-dihydrobenzo[1,4]dioxinyl or benzo[1,3]dioxolyl; each monocyclic and bicyclic heterocycle may optionally be substituted with 1, 2 or 3 alkyl or Ar-carbonyl substituents ; and
- halo: is a substituent selected from the group of fluoro, chloro and bromo.

In a third interesting embodiment the compounds of formula (Ia) or (Ib) or any subgroup thereof as mentioned hereinbefore as interesting embodiment are those compounds according to either formula (Ia) and (Ib) wherein R¹ is hydrogen, halo, Ar, alkyl or alkyloxy; preferably, R¹ is halo; more preferably, R¹ is bromo.

In a fourth interesting embodiment the compounds of formula (Ia) or (Ib) or any subgroup thereof as mentioned hereinbefore as interesting embodiment are those compounds according to either formula (Ia) and (Ib) wherein p is equal to 1 and R¹ is different from hydrogen.

In a fifth interesting embodiment the compounds of formula (Ia) or (Ib) or any subgroup thereof as mentioned hereinbefore as interesting embodiment are those compounds according to either formula (Ia) and (Ib) wherein R² is hydrogen, alkyloxy or alkylthio; preferably, R² is alkyloxy, in particular C₁₋₄alkyloxy; more preferably, R² is methyloxy.

C₁₋₄alkyl is a straight or branched saturated hydrocarbon radical having from 1 to 4 carbon atoms such as for example methyl, ethyl, propyl, 2-methyl-ethyl and the like.

In a sixth interesting embodiment the compounds of formula (Ia) or (Ib) or any subgroup thereof as mentioned hereinbefore as interesting embodiment are those compounds according to either formula (Ia) and (Ib) wherein R³ is naphthyl, phenyl or thienyl, each optionally substituted with 1 or 2 substituents, that substituent preferably being a halo or haloalkyl, most preferably being a halo; preferably, R³ is naphthyl or phenyl, each optionally substituted with halo, preferably 3-fluoro; more preferably, R³ is naphthyl or phenyl; most preferably, R³ is naphthyl.

In a seventh interesting embodiment the compounds of formula (Ia) or (Ib) or any subgroup thereof as mentioned hereinbefore as interesting embodiment are those compounds according to either formula (Ia) and (Ib) wherein q is equal to zero, 1 or 2; preferably, q is equal to 1.

In an eighth interesting embodiment the compounds of formula (Ia) or (Ib) or any subgroup thereof as mentioned hereinbefore as interesting embodiment are those compounds according to either formula (Ia) and (Ib) wherein R⁴ and R⁵ each independently are hydrogen or alkyl, in particular hydrogen or C₁₋₄alkyl, more in particular C₁₋₄alkyl; preferably hydrogen, methyl or ethyl; most preferably methyl. C₁₋₄alkyl is a straight or branched saturated hydrocarbon radical having from 1 to 4 carbon atoms such as for example methyl, ethyl, propyl, 2-methyl-ethyl and the like.

In a ninth interesting embodiment the compounds of formula (Ia) or (Ib) or any subgroup thereof as mentioned hereinbefore as interesting embodiment are those compounds according to either formula (Ia) and (Ib) wherein R⁴ and R⁵ together and including the N to which they are attached form a radical selected from the group of imidazolyl, triazolyl, piperidinyl, piperazinyl and thiomorpholinyl, optionally substituted with alkyl, halo, haloalkyl, hydroxy, alkyloxy, alkylthio, alkyloxyalkyl or alkylthioalkyl, preferably substituted with alkyl, most preferably substituted with methyl or ethyl.

In a tenth interesting embodiment the compounds of formula (Ia) or (Ib) or any subgroup thereof as mentioned hereinbefore as interesting embodiment are those compounds according to either formula (Ia) and (Ib) wherein R⁶ is hydrogen, alkyl or halo; preferably, R⁶ is hydrogen.

In an eleventh interesting embodiment the compounds of formula (Ia) or (Ib) or any subgroup thereof as mentioned hereinbefore as interesting embodiment are those compounds according to either formula (Ia) and (Ib) wherein r is 1 or 2.

In a twelfth interesting embodiment the compounds of formula (Ia) or (Ib) or any subgroup thereof as mentioned hereinbefore as interesting embodiment are those compounds according to either formula (Ia) and (Ib) wherein R⁷ is hydrogen or methyl; preferably R⁷ is hydrogen.

In a thirteenth interesting embodiment the compounds of formula (Ia) or (Ib) or any subgroup thereof as mentioned hereinbefore as interesting embodiment are those compounds according to either formula (Ia) and (Ib) wherein, for compounds according to Formula (Ib) only, R⁸ is alkyl, preferably methyl, and R⁹ is oxygen.

In a fourteenth interesting embodiment the compounds of formula (Ia) or (Ib) or any subgroup thereof as mentioned hereinbefore as interesting embodiment are those compounds according to either formula (Ia) and (Ib) wherein the compound is a compound according to formula (Ia), a pharmaceutically acceptable acid or base addition salt thereof, a quaternary amine thereof, a *N-*oxide thereof, a tautomeric form thereof or a stereochemically isomeric form thereof.

A fifteenth interesting embodiment of the compounds of formula (Ia) or (Ib) are the compounds according to formula (Ia), the pharmaceutically acceptable acid or base addition salts thereof, the quaternary amines thereof, the stereochemically isomeric forms thereof, the tautomeric forms thereof or the *N-*oxide forms thereof, in which R¹ is hydrogen, halo, Ar, alkyl or alkyloxy; p = 1; R² is hydrogen, alkyloxy or alkylthio; R³ is naphthyl, phenyl or thienyl, each optionally substituted with 1 or 2 substituents selected from the group of halo and haloalkyl; q = 0, 1, 2 or 3; R⁴ and R⁵ each independently are hydrogen or alkyl or R⁴ and R⁵ together and including the N to which they are attached form a radical selected from the group of imidazolyl, triazolyl, piperidinyl, piperazinyl and thiomorpholinyl; R⁶ is hydrogen, alkyl or halo; r is equal to 1 and R⁷ is hydrogen.

A sixteenth interesting embodiment of the compounds of formula (Ia) or (Ib) are the compounds of formula (Ia) or (Ib) or any subgroup thereof as mentioned hereinbefore as interesting embodiment or the pharmaceutically acceptable acid or base addition salts thereof.

A seventeenth interesting embodiment of the compounds of formula (Ia) or (Ib) are the compounds of formula (Ia) or (Ib) or any subgroup thereof as mentioned hereinbefore as interesting embodiment or the quaternary amines thereof.

An eighteenth interesting embodiment of the compounds of formula (Ia) or (Ib) are the compounds of formula (Ia) or (Ib) or any subgroup thereof as mentioned hereinbefore as interesting embodiment or the N-oxides thereof.

A nineteenth interesting embodiment of the compounds of formula (Ia) or (Ib) are the compounds of formula (Ia) or (Ib) or any subgroup thereof as mentioned hereinbefore as interesting embodiment or the stereochemically isomeric forms thereof.

A twentieth interesting embodiment of the compounds of formula (Ia) or (Ib) are the compounds of formula (Ia) or (Ib) or any subgroup thereof as mentioned hereinbefore as interesting embodiment.

Preferably, in the compounds of formula (Ia) and (Ib) or any subgroup thereof as mentioned hereinbefore as interesting embodiment, the term-"alkyl" represents C₁₋₆alkyl wherein C₁₋₆alkyl is a straight or branched saturated hydrocarbon radical having from 1 to 6 carbon atoms such as for example methyl, ethyl, propyl, 2-methyl-ethyl, pentyl, hexyl and the like.

Preferably, in the compounds of formula (Ia) and (Ib) or any subgroup thereof as mentioned hereinbefore as interesting embodiment, the term "haloalkyl" represents polyhaloC₁₋₆alkyl which is defined as mono- or polyhalosubstituted C₁₋₆alkyl, for example, methyl with one or more fluoro atoms, for example, difluoromethyl or trifluoromethyl, 1,1-difluoro-ethyl and the like. In case more than one halogen atoms are attached to an alkyl group within the definition of polyhaloC₁₋₆alkyl, they may be the same or different. C₁₋₆alkyl is a straight or branched saturated hydrocarbon radical having from 1 to 6 carbon atoms such as for example methyl, ethyl, propyl, 2-methyl-ethyl, pentyl, hexyl and the like.

Preferably, the compound is selected from :
O 1-(6-bromo-2-methoxy-quinolin-3-yl)-2-(3,5-difluoro-phenyl)-4-dimethylamino-1-phenyl-butan-2-ol ;
O 1-(6-bromo-2-methoxy-quinolin-3-yl)-4-dimethylamino-2-naphthalen-1-yl-1-phenyl-butan-2-ol corresponding to 6-bromo-α-[2-(dimethylamino)ethyl]-2-methoxy-α-1-naphthalenyl-β-phenyl-3-quinolineethanol;
O 1-(6-bromo-2-methoxy-quinolin-3-yl)-2-(2,5-difluoro-phenyl)-4-dimethylamino-1-phenyl-butan-2-ol;
O 1-(6-bromo-2-methoxy-quinolin-3-yl)-2-(2,3-difluoro-phenyl)-4-dimethylamino-1-phenyl-butan-2-ol;
O 1-(6-bromo-2-methoxy-quinolin-3-yl)-4-dimethylamino-2-(2-fluoro-phenyl)-1-phenyl-butan-2-ol;
O 1-(6-bromo-2-methoxy-quinolin-3-yl)-4-dimethylamino-2-naphthalen-1-yl-1-*p-*tolyl-butan-2-ol ;
O 1-(6-bromo-2-methoxy-quinolin-3-yl)-4-methylamino-2-naphthalen-1-yl-1-phenyl-butan-2-ol ;
O 1-(6-bromo-2-methoxy-quinolin-3-yl)-4-dimethylamino-2-(3-fluoro-phenyl)-1-phenyl-butan-2-ol; and
O 1-(6-bromo-2-methoxy-quinolin-3-yl)-4-dimethylamino-2-phenyl-1-phenyl-butan-2-ol;
a pharmaceutically acceptable acid or base addition salt thereof, a *N-*oxide thereof, a tautomeric form thereof or a stereochemically isomeric form thereof.

More preferably, the compound is
O 1-(6-bromo-2-methoxy-quinolin-3-yl)-2-(2,3-difluoro-phenyl)-4-dimethylamino-1-phenyl-butan-2-ol ; or
O 1-(6-bromo-2-methoxy-quinolin-3-yl)-4-dimethylamino-2-naphthalen-1-yl-1-phenyl-butan-2-ol corresponding to 6-bromo-α-[2-(dimethylamino)ethyl]-2-methoxy-α-1-naphthalenyl-β-phenyl-3-quinolineethanol;
a pharmaceutically acceptable acid or base addition salt thereof, a *N-*oxide thereof, a tautomeric form thereof or a stereochemically isomeric form thereof

Even more preferably, the compound is 1-(6-bromo-2-methoxy-quinolin-3-yl)-4-dimethylamino-2-naphthalen-1-yl-1-phenyl-butan-2-ol, a pharmaceutically acceptable acid or base addition salt thereof, a *N-*oxide thereof, or a stereochemically isomeric form thereof.

An alternative chemical name for 1-(6-bromo-2-methoxy-quinolin-3-yl)-4-dimethylamino-2-naphthalen-1-yl-1-phenyl-butan-2-ol is 6-bromo-α-[2-(dimethylamino)ethyl]-2-methoxy-α-1-naphthalenyl-β-phenyl-3-quinolineethanol. Said compound can also be represented as follows:

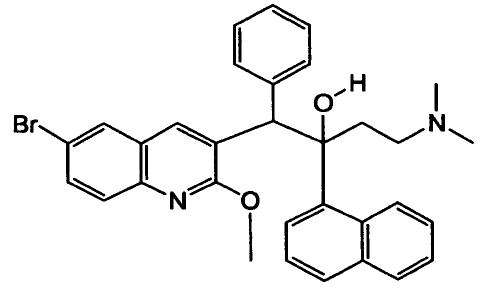

Further preferably, the compound is one of the following:
6-bromo-α-[2-(dimethylamino)ethyl]-2-methoxy-α-1-naphthalenyl-β-phenyl-3-quinolineethanol, or a pharmaceutically acceptable acid addition salt thereof; or
6-bromo-α-[2-(dimethylamino)ethyl]-2-methoxy-α-1-naphthalenyl-β-phenyl-3-quinolineethanol, or a stereochemically isomeric form thereof; or
6-bromo-α-[2-(dimethylamino)ethyl]-2-methoxy-α-1-naphthalenyl-β-phenyl-3-quinolineethanol, or a *N-*oxide form thereof; or
a mixture, in particular a racemic mixture, of (αS, βR)-6-bromo-α-[2-(dimethylamino)ethyl]-2-methoxy-α-1-naphthalenyl-β-phenyl-3-quinolineethanol and (αR, βS)-6-bromo-α-[2-(dimethylamino)ethyl]-2-methoxy-α-1-naphthalenyl-β-phenyl-3-quinolineethanol, or a pharmaceutically acceptable acid addition salt thereof, or a stereochemically isomeric forms thereof; i.e. compound 14 (diastereoisomer A); or (αS,βR)-6-bromo-α-[2-(dimethylamino)ethyl]-2-methoxy-α-1-naphthalenyl-β-phenyl-3-quinolineethanol, i.e. compound 12, or a pharmaceutically acceptable acid addition salt thereof; or
(αS,βR)-6-bromo-α-[2-(dimethylamino)ethyl]-2-methoxy-α-1-naphthalenyl-β-phenyl-3-quinolineethanol, i.e. compound 12.

The most preferred compound is (αS, βR)-6-bromo-α-[2-(dimethylamino)ethyl]-2-methoxy-α-1-naphthalenyl-β-phenyl-3-quinolineethanol which corresponds to (1R,2S)-1-(6-bromo-2-methoxy-quinolin-3-yl)-4-dimethylamino-2-naphthalen-1-yl-1-phenyl-butan-2-ol. Said compound can also be represented as follows :

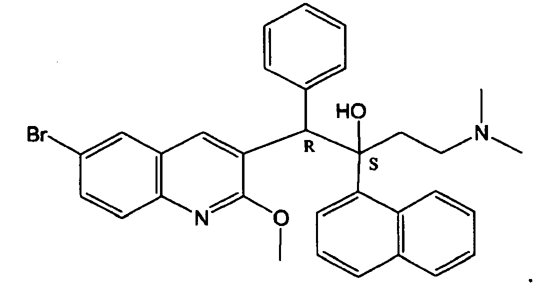

Another interesting group of compounds is the following: compounds 12, 71, 174, 75, 172, 79 and 125 as described hereinafter in Tables 1 to 6; in particular compounds 12, 71, 174, 75, 172 and 79 or compounds 12, 71, 75, 172 and 125; more in particular compounds 12, 71, 174 and 75 or compounds 12, 71, 75 and 172; even more in particular compounds 12, 71 and 174 or compounds 12, 71 and 75; a pharmaceutically acceptable acid or base addition salt thereof, a *N-*oxide thereof, a tautomeric form thereof or a stereochemically isomeric form thereof.

The compounds of formula (Ia) and (Ib) can be prepared according to the methods described in WO 2004/011436.

In general, the compounds according to the invention can be prepared by a succession of steps, each of which is known to the skilled person.

In particular, the compounds according to formula (Ia) can be prepared by reacting an intermediate compound of formula (II) with an intermediate compound of formula (III) according to the following reaction scheme (1) : 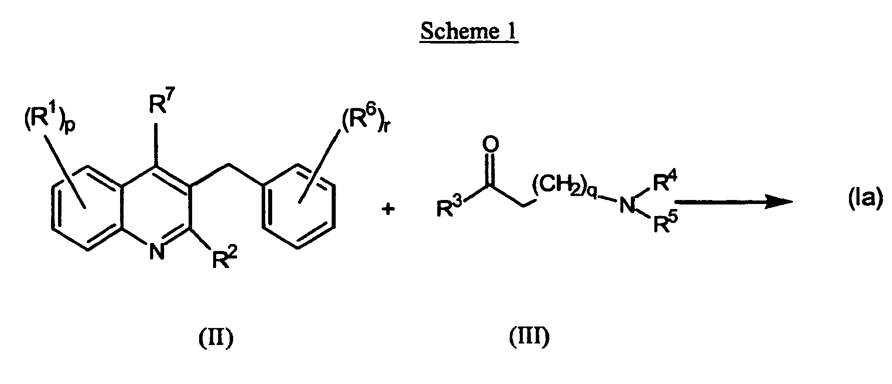 using BuLi in a mixture of diisopropyl amine and tetrahydrofuran, and wherein all variables are defined as in formula (Ia). Stirring may enhance the rate of the reaction. The reaction may conveniently be carried out at a temperature ranging between -20 and -70°C.

The same reaction procedure can be used to synthesize compounds of formula (Ib).

The starting materials and the intermediate compounds of formula (II) and (III) are compounds that are either commercially available or may be prepared according to conventional reaction procedures generally known in the art. For example, intermediate compounds of formula (II-a) may be prepared according to the following reaction scheme (2): 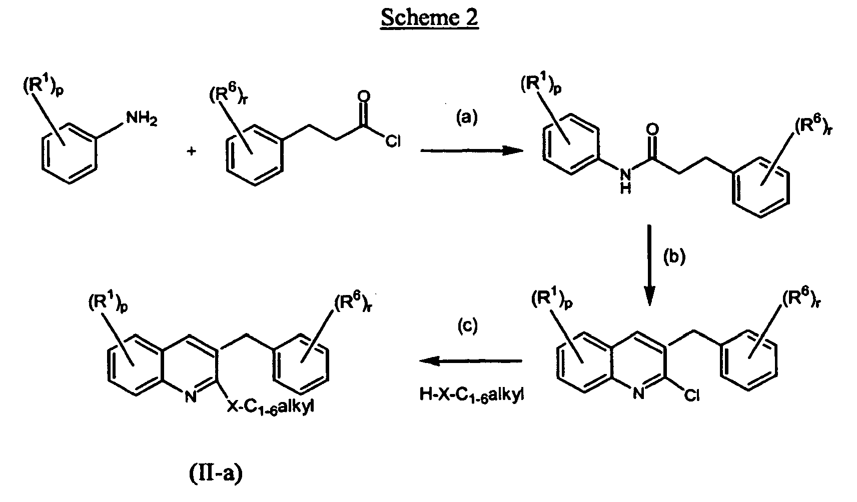 wherein all variables are defined as in formula (Ia). Reaction scheme (2) comprises step (a) in which an appropriately substituted aniline is reacted with an appropriate acylchloride such as 3-phenylpropionyl chloride, 3-fluorobenzenepropanoyl chloride or *p-*chlorobenzenepropanoyl chloride, in the presence of a suitable base, such as triethylamine and a suitable reaction-inert solvent, such as methylene chloride or ethylene dichloride. The reaction may conveniently be carried out at a temperature ranging between room temperature and reflux temperature. In a next step (b) the adduct obtained in step (a) is reacted with phosphoryl chloride (POCl₃) in the presence of *N*,*N-*dimethylformamide (Vilsmeier-Haack formylation followed by cyclization). The reaction may conveniently be carried out at a temperature ranging between room temperature and reflux temperature. In a next step (c) a specific R²-group, wherein R² is for example an C₁₋₆alkyloxy or C₁₋₆alkylthio radical is introduced by reacting the intermediate compound obtained in step (b) with a compound H-X-C₁₋₆alkyl wherein X is S or O.

Intermediate compounds according to formula (II-b) may be prepared according to the following reaction scheme (3), wherein in a first step (a) a substituted indole-2,3-dione is reacted with a substituted 3-phenylpropionaldehyde in the presence of a suitable base such as sodium hydroxide (Pfitzinger reaction), after which the resulting carboxylic acid compound is decarboxylated in a next step (b) at high temperature in the presence of a suitable reaction-inert solvent such as diphenylether.

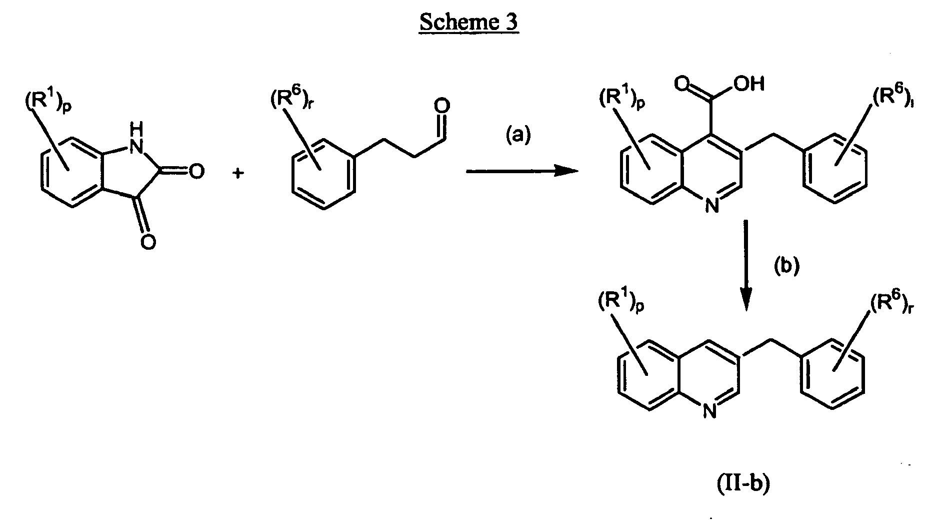

It is evident that in the foregoing and in the following reactions, the reaction products may be isolated from the reaction medium and, if necessary, further purified according to methodologies generally known in the art, such as extraction, crystallization and chromatography. It is further evident that reaction products that exist in more than one enantiomeric form, may be isolated from their mixture by known techniques, in particular preparative chromatography, such as preparative HPLC. Typically, compounds of formula (I) may be separated into their isomeric forms.

The intermediate compounds of formula (III) are compounds that are either commercially available or may be prepared according to conventional reaction procedures generally known in the art. For example, intermediate compounds of formula (III-a) in which R³ is Ar substituted with s substituents R¹⁰, wherein each R¹⁰ is independently selected from the group of hydroxy, halo, cyano, nitro, amino, mono- or di(C₁₋₆alkyl)amino, C₁₋₆alkyl, polyhaloC₁₋₆alkyl, C₁₋₆alkyloxy, polyhaloC₁₋₆alkyloxy, carboxyl, C₁₋₆alkyloxycarbonyl, aminocarbonyl, morpholinyl and mono- or di(C₁₋₆alkyl)aminocarbonyl and s is an integer equal to zero, 1, 2 or 3, may be prepared according to the following reaction scheme (4):

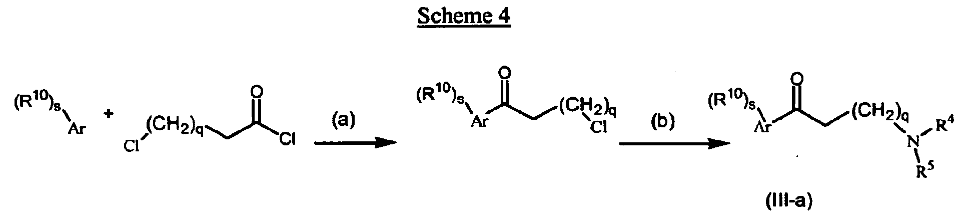

Reaction scheme (4) comprises step (a) in which an appropriately substituted Ar, in particular an appropriately substituted phenyl, is reacted by Friedel-Craft reaction with an appropriate acylchloride such as 3-chloropropionyl chloride or 4-chlorobutyryl chloride, in the presence of a suitable Lewis acid, such as for example AlCl₃, FeCl₃, SnCl₄, TiCl₄ or ZnCl₂ and a suitable reaction-inert solvent, such as methylene chloride or ethylene dichloride. The reaction may conveniently be carried out at a temperature ranging between room temperature and reflux temperature. In a next step (b) an amino group (-NR⁴R⁵) is introduced by reacting the intermediate compound obtained in step (a) with an appropriate primary or secondary amine.

As for the interpretation of the present invention, latent TB, dormant TB or persistent TB are the same (TB stands for tuberculosis).

As already stated above, the compounds of formula (Ia) and (Ib) can be used to treat latent TB. The exact dosage and frequency of administration of the present compounds depends on the particular compound of formula (Ia) and (Ib) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention.

The compounds of the present invention may be administered in a pharmaceutically acceptable form optionally in a pharmaceutically acceptable carrier.

The pharmaceutical compositions may have various pharmaceutical forms for administration purposes. As appropriate compositions there may be cited all compositions usually employed for systemically administering drugs. To prepare the pharmaceutical compositions, an effective amount of the particular compounds, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral unit dosage forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations.

Depending on the mode of administration, the pharmaceutical composition will preferably comprise from 0.05 to 99 % by weight, more preferably from 0.1 to 70 % by weight of the active ingredient, and, from 1 to 99.95 % by weight, more preferably from 30 to 99.9 weight % of a pharmaceutically acceptable carrier, all percentages being based on the total composition.

The pharmaceutical composition may additionally contain various other ingredients known in the art, for example, a lubricant, stabilising agent, buffering agent, emulsifying agent, viscosity-regulating agent, surfactant, preservative, flavouring or colorant.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof. The daily dosage of the compound according to the invention will, of course, vary with the compound employed, the mode of administration, the treatment desired and the mycobacterial disease indicated. However, in general, satisfactory results will be obtained when the compound according to the invention is administered at a daily dosage not exceeding 1 or 2 gram, e.g. in the range from 10 to 50 mg/kg body weight.

### EXPERIMENTAL PART

As already stated above, the compounds of formula (Ia) and (Ib) and their preparation is described in WO 2004/011436.

Of some compounds the absolute stereochemical configuration of the stereogenic carbon atom(s) therein was not experimentally determined. In those cases the stereochemically isomeric form which was first isolated is designated as "A" and the second as "B", without further reference to the actual stereochemical configuration. However, said "A" and "B" isomeric forms can be unambiguously characterized by a person skilled in the art, using art-known methods such as, for example, X-ray diffraction.

In case "A" and "B" are stereoisomeric mixtures, they can be further separated whereby the respective first fractions isolated are designated "A1" respectively "B1" and the second as "A2" respectively "B2", without further reference to the actual stereochemical configuration. However, said "A1, A2" and "B1, B2" isomeric forms can be unambiguously characterized by a person skilled in the art, using art-known methods such as, for example, X-ray diffraction.

The present compounds (see Tables 1 to 6) are numbered in conformity with the compounds of WO 2004/011436 and can be prepared according to the methods described in WO 2004/011436. The Ex. Nr. in the below Tables refer to the Example numbers of WO 2004/011436 indicating according to which procedure the compounds can be prepared.

In particular, the preparation of compounds 12, 13, 12a, 13a, 14 and 15 are described below in detail.

Hereinafter, "DMF" is defined as *N*,*N-*dimethylformamide, "THF" is defined as tetrahydrofuran, "DIPE" is defined as diisopropylether.

### Preparation of the intermediate compounds

### Example A1

### Preparation of intermediate compound 1

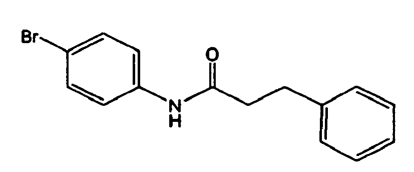

Benzenepropanoylchloride (0.488 mol) was added dropwise at room temperature to a solution of 4-bromobenzenamine (0.407 mol) in Et₃N (70ml) and CH₂Cl₂ (700ml) and the mixture was stirred at room temperature overnight. The mixture was poured out into water and concentrated NH₄OH, and extracted with CH₂Cl₂. The organic layer was dried (MgSO₄) filtered, and the solvent was evaporated. The residue was crystallized from diethyl ether. The residue (119.67g) was taken up in CH₂Cl₂ and washed with HCl 1N. The organic layer was dried (MgSO₄) filtered, and the solvent was evaporated. Yield: 107.67g of intermediate compound 1.

### Example A2

### Preparation of intermediate compound 2

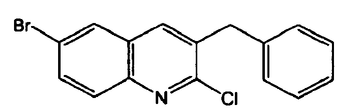

The reaction was carried out twice. POCl₃ (1.225 mol) was added dropwise at 10°C to *N*,*N-*dimethylformamide (DMF) (0.525 mol). Then intermediate compound 1 (prepared according A1) (0.175 mol) was added at room temperature . The mixture was stirred overnight at 80°C, poured out on ice and extracted with CH₂Cl₂. The organic layer was dried (MgSO₄) filtered, and the solvent was evaporated. The product was used without further purification. Yield : 77.62g of intermediate compound 2 (67%).

### Example A3

### Preparation of intermediate compound 3

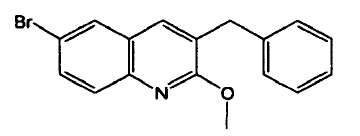

A mixture of intermediate compound 2 (prepared according to A2) (0.233 mol) in CH₃ONa (30%) in methanol (222.32 ml) and methanol (776ml) was stirred and refluxed overnight, then poured out on ice and extracted with CH₂Cl₂. The organic layer was separated, dried (MgSO₄), filtered and the solvent was evaporated. The residue was purified by column chromatography over silica gel (eluent: CH₂Cl₂/cyclohexane 20/80 and then 100/0; 20-45µm). The pure fractions were collected and the solvent was evaporated. Yield : 25g of intermediate compound 3 (Yield=33%; mp.84°C) as a white powder.

### Preparation of final compounds 12, 13, 12a, 13a, 14 and 15

### Preparation of final compounds 12, 13, 12a, 13a, 14 and 15

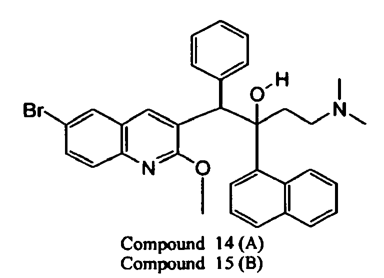

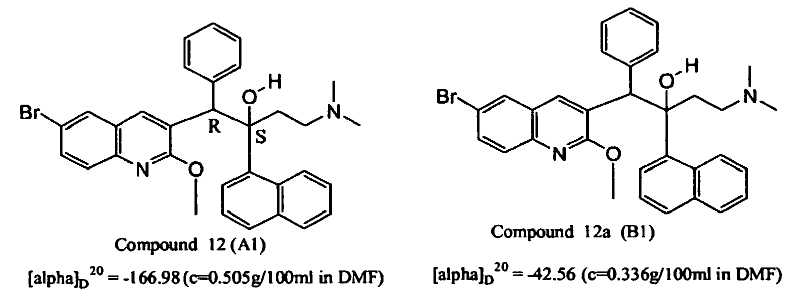

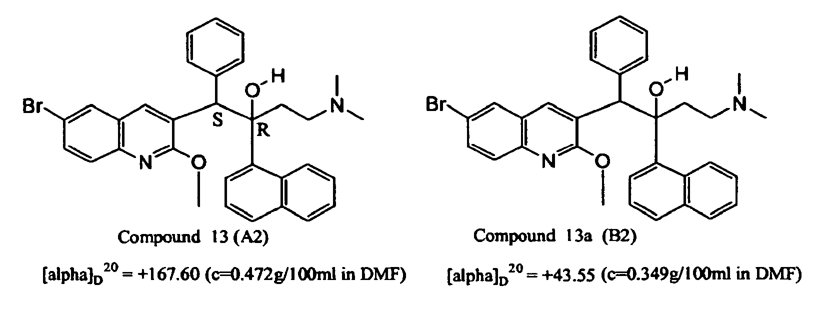

nBuLi 1.6M (0.05 mol) was added slowly at -20°C under N₂ flow to a solution of *N*-(1-methylethyl)-2-propanamine (0.05 mol) in tetrahydrofuran (THF) (80ml). The mixture was stirred at -20°C for 15 minutes, then cooled to -70°C . A solution of intermediate compound 3 (prepared according to A3 described above) (0.046 mol) in THF (150ml) was added slowly. The mixture was stirred at -70°C for 30 minutes. A solution of 0.055 mol of 3-(dimethylamino)-1-(1-naphthyl)-1-propanone in THF (120ml) was added slowly. The mixture was stirred at -70°C for 3 hours, hydrolyzed at -30°C with ice water and extracted with EtOAc. The organic layer was separated, dried (MgSO₄), filtered, and the solvent was evaporated. The residue (29g) was purified by column chromatography over silica gel (eluent: CH₂Cl₂/CH₃OH/NH₄OH; 99.5/0.5/0.1; 15-35µm). Two fractions were collected and the solvent was evaporated, yielding 3g of fraction 1 and 4.4g of fraction 2 . Fraction 1 and 2 were crystallized separately from DIPE. The precipitate was filtered off and dried, yielding 2.2g of diastereoisomer A, i.e. final compound 14 (Yield: 9%; mp.210°C) as a white solid and 4g of diastereoisomer B, i.e. final compound 15 (Yield: 16%; mp.244°C) as a white solid. To obtain the corresponding enantiomers, diastereoisomer A (final compound 14) was purified by chiral chromatography over silica gel (chiralpack AD) (eluent: hexane/EtOH; 99.95/0.05). Two fractions were collected and the solvent was evaporated. Yield: 0.233g of enantiomer A1 (final compound 12) (mp. 118°C, [α]_{D}²⁰ = -166.98° (c = 0.505 g/100 ml in DMF)) as a white solid and 0.287g of enantiomer A2 (final compound 13) (mp. 120°C, [α]_{D}²⁰ =+167.60° (c = 0.472 g/100 ml in DMF)) as a white solid. Enantiomer A1 was crystallised from EtOH to give a white solid: mp. 184°C, [α]_{D}²⁰ = -188.71°(c= 0.621 g/100ml in DMF). Crystallization of enantiomer A2 from EtOH gave a solid with mp. of 175°C.
0.2g of diastereoisomer B (final compound 15) was purified by chiral chromatography over silica gel (chiralpack AD) (eluent: EtOH/iPrOH/*N-*ethyl-ethanamine; 50/50/0.1). Two fractions were collected and the solvent was evaporated. Yield: 78.2mg of enantiomer B1 and 78.8mg of enantiomer B2. Enantiomer B1 was purified by column chromatography over silica gel (eluent: CH₂Cl₂/CH₃OH/NH₄OH; 99/1/0.1; 15-40µm). One fraction was collected and the solvent was evaporated. Yield: 57mg of enantiomer B1 (final compound 12a) ([α]_{D}²⁰ = -42.56°(c = 0.336 g/100 ml in DMF)). Enantiomer B2 was purified by column chromatography over silica gel (eluent: CH₂Cl₂/CH₃OH/NH₄OH; 99/1/0.1; 15-40µm). One fraction was collected and the solvent was evaporated. Yield: 53mg of enantiomer B2 (final compound 13a) ([α]_{D}²⁰ = + 43.55°(c = 0.349 g/100ml in DMF)).

Tables 1 to 6 list compounds of formula (Ia) and (Ib).

**Table 1 :**

| | | | | | | |
|---|---|---|---|---|---|---|
| 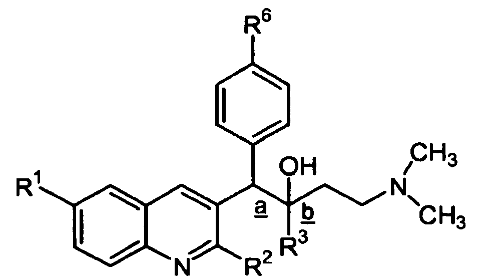 | | | | | | |

| Comp. nr. | Ex. nr. | R¹ | R² | R³ | R⁶ | Stereochemistry and melting points |
|---|---|---|---|---|---|---|
| 1 | B1 | Br | OCH₃ | phenyl | H | (A1); 194°C |
| 2 | B1 | Br | OCH₃ | phenyl | H | (A2); 191°C |
| 3 | B1 | Br | OCH₃ | phenyl | H | (A); 200°C |
| 4 | B1 | Br | OCH₃ | phenyl | H | (B); 190°C |
| 16 | B1 | Br | OCH₃ | 4-chlorophenyl | H | (A); 200°C |
| 17 | B1 | Br | OCH₃ | 4-chlorophenyl | H | (B); 190°C |
| 20 | B1 | Br | OCH₃ | 2-thienyl | H | (A); 96°C |
| 21 | B1 | Br | OCH₃ | 2-thienyl | H | (B); 176°C |
| 22 | B1 | CH₃ | OCH₃ | phenyl | H | (A); 148°C |
| 23 | B1 | CH₃ | OCH₃ | phenyl | H | (B); 165°C |
| 24 | B1 | Br | OCH₃ | 3-thienyl | H | (A); 162°C |
| 25 | B1 | Br | OCH₃ | 3-thienyl | H | (B); 160°C |
| 26 | B1 | phenyl | OCH₃ | phenyl | H | (A); 174°C |
| 27 | B1 | phenyl | OCH₃ | phenyl | H | (B); 192°C |
| 28 | B1 | F | OCH₃ | phenyl | H | (A) 190°C |
| 29 | B1 | F | OCH₃ | phenyl | H | (B); 166°C |
| 30 | B1 | Cl | OCH₃ | phenyl | H | (A); 170°C |
| 31 | B1 | Cl | OCH₃ | phenyl | H | (B); 181°C |
| 32 | B1 | Br | SCH₃ | phenyl | H | (A); 208°C |
| 33 | B1 | Br | SCH₃ | phenyl | H | (B); 196°C |
| 34 | B1 | OCH₃ | OCH₃ | phenyl | H | (A); 165°C |
| 35 | B1 | OCH₃ | OCH₃ | phenyl | H | (B); 165°C |
| 36 | B1 | Br | OCH₃ | phenyl | Cl | (A); 197°C |
| 37 | B1 | Br | OCH₃ | phenyl | Cl | (B); 221°C |
| 38 | B9 | Br | OCH₃ | 3-fluorophenyl | H | (A); 198°C |
| 39 | B9 | Br | OCH₃ | 3-fluorophenyl | H | (B); 207°C |
| 108 | B9 | Br | OCH₃ | 3-fluorophenyl | H | (A1); 160°C |
| 109 | B9 | Br | OCH₃ | 3-fluorophenyl | H | (A2); 156°C |
| 40 | B1 | H | OCH₃ | phenyl | H | (A); 152°C |
| 41 | B1 | H | OCH₃ | phenyl | H | (B); 160°C |
| 42 | B1 | H | OCH₃ | CH₃ | H | (A); 40°C |
| 43 | B1 | H | OCH₃ | CH₃ | H | (B); 120°C |
| 59 | B1 | Br | OH | phenyl | H | (A); >260°C |
| 60 | B1 | Br | OH | phenyl | H | (B); 215°C |
| 5 | B2 | Br | OCH₂CH₃ | phenyl | H | (A); 162°C |
| 6 | B2 | Br | OCH₂CH₃ | phenyl | H | (B); 74°C |
| 7 | B3 | Br | H | phenyl | H | (A); 98°C |
| 8 | B3 | Br | H | phenyl | H | (B); 180°C |
| 12 | B7 | Br | OCH₃ | 1-naphthyl | H | (A1); 118°C (foam); a=R, b=S; [alpha]_{D}²⁰=-166.98 (c=0.505g/100ml in DMF) |
| 13 | B7 | Br | OCH₃ | 1-naphthyl | H | (A2); 120°C (foam); a=S; b=R; [alpha]_{D}²⁰=+167.60 (c=0.472g/100ml in DMF) |
| 12a | B7 | Br | OCH₃ | 1-naphthyl | H | (B1); [α]_{D}²⁰ =-42.56 (c = 0.336 g/100 ml in DMF) |
| 13a | B7 | Br | OCH₃ | 1-naphthyl | H | (B2); [α]_{D}²⁰ =+43.55 (c = 0.349 g/100 ml in DMF |
| 14 | B7 | Br | OCH₃ | 1-naphthyl | H | (A); 210°C |
| 15 | B7 | Br | OCH₃ | 1-naphthyl | H | (B); 244°C |
| 45 | B7 | Br | OCH₃ | 2-naphthyl | H | (A); 262°C 162°C |
| 46 | B7 | Br | OCH₃ | 2-naphthyl | H | (B); |
| 67 | B8 | Br | OCH₃ | 2,5-difluorophenyl | H | (A); 60°C |
| 68 | B8 | Br | OCH₃ | 2,5-difluorophenyl | H | (B); 208°C |
| 110 | B8 | Br | OCH₃ | 2,5-difluorophenyl | H | (A1); 167°C |
| 111 | B8 | Br | OCH₃ | 2,5-difluorophenyl | H | (A2); oil |
| 69 | B1 | Br | OCH₃ | 2-fluorophenyl | H | (A); oil |
| 70 | B1 | Br | OCH₃ | 2-fluorophenyl | H | (B); oil |
| 71 | B1 | Br | OCH₃ | 1-naphthyl | CH₃ | points (A); 174°C |
| 72 | B1 | Br | OCH₃ | 1-naphthyl | CH₃ | (B); 178°C |
| 73 | B1 | Br | OCH₃ | 1-naphthyl | Cl | (B); 174°C |
| 74 | B1 | Br | OCH₃ | 1-naphthyl | Cl | (A); 110°C |
| 75 | B1 | Br | OCH₃ | 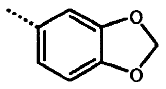 | H | (A); 196°C |
| 76 | B1 | Br | OCH₃ | 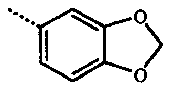 | H | (B); 130°C |
| 77 | B1 | Br | OCH₃ | 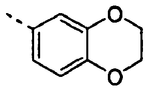 | H | (A); 202°C |
| 78 | B1 | Br | OCH₃ | 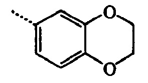 | H | (B); 202°C |
| 79 | B1 | Br | 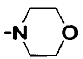 | 1-naphthyl | H | (A); >250°C |
| 80 | B1 | Br | OCH₃ | 4-cyanophenyl | H | (A); 224°C |
| 81 | B1 | Br | OCH₃ | 4-cyanophenyl | H | (B); 232°C |
| 82 | B1 | CH₃ | OCH₃ | 1-naphthyl | H | (A); 202°C |
| 83 | B1 | CH₃ | OCH₃ | 1-naphthyl | H | (B); 198°C |
| 84 | B1 | phenyl | OCH₃ | 1-naphthyl | H | (A); 248°C |
| 85 | B1 | phenyl | OCH₃ | 1-naphthyl | H | (B); 214°C |
| 86 | B1 | Br | OCH₃ | 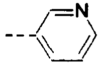 | H | (A); 184°C |
| 87 | B1 | Br | OCH₃ | 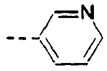 | H | (B); 186°C |
| 88 | B1 | Br | SCH₃ | 1-naphthyl | H | (A); 240°C |
| 89 | B1 | Br | OCH₃ | 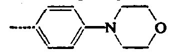 | H | (A); 236°C |
| 90 | B1 | Br | OCH₃ | 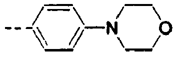 | H | (B); 206°C |
| 91 | B1 | H | OCH₃ | 1-naphthyl | H | (A); 178°C |
| 92 | B1 | H | OCH₃ | 1-naphthyl | H | (B); 160°C |
| 93 | B1 | H | OCH₃ | 3-fluorophenyl | H | (A); 178°C |
| 94 | B1 | H | OCH₃ | 3-fluorophenyl | H | (B); 182°C |
| 95 | B1 | Br | OCH₃ | 2-phenytethyl | H | (A); 178°C |
| 96 | B1 | Br | OCH₃ | 2-phenylethyl | H | (B); 146°C |
| 97 | nr. B1 | OCH₃ | OCH₃ | 1-naphthyl | H | points (A); 168°C |
| 98 | B1 | OCH₃ | OCH₃ | 1-naphthyl | H | (B); 154°C |
| 113 | B14 | Br | OCH₃ | 2,3,-difluorophenyl | H | (A); 128°C |
| 114 | B14 | Br | OCH₃ | 2,3-difluorophenyl | H | (B); 213°C |
| 115 | B15 | Br | OCH₃ | 3,5-difluorophenyl | H | (A); 192°C |
| 116 | B15 | Br | OCH₃ | 3,5-difluorophenyl | H | (B); 224°C |
| 117 | B15 | Br | OCH₃ | 3,5-difluorophenyl | H | (A1); 161°C |
| 118 | B15 | Br | OCH₃ | 3,5-difluorophenyl | H | (A2); 158°C |
| 119 | B7 | Cl | OCH₃ | 1-naphthyl | H | (A); 212°C |
| 120 | B7 | Cl | OCH₃ | 1-naphthyl | H | (B); 236°C |
| 122 | B7 | Br | OCH₃ | 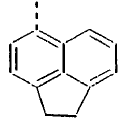 | H | (B); 227°C |
| 127 | B7 | Br | OCH₃ | 5-bromo-2-naphthyl | H | (A); 226°C |
| 130 | B7 | Br | OCH₃ | 5-bromo-2-naphthyl | H | (B); 220°C |
| 131 | B1 | Br | OCH₃ | 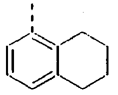 | H | (A); 206°C |
| 134 | B9 | OCH₃ | OCH₃ | 3-fluorophenyl | H | (A); 172°C |
| 135 | B9 | OCH₃ | OCH₃ | 3-fluorophenyl | H | (B); 182°C |
| 143 | B7 | Br | OCH₃ | 3-bromo-1-naphthyl | H | (A); 234°C |
| 150 | B7 | Br | OCH₃ | 3-bromo-1-naphthyl | H | (B); 212°C |
| 159 | B8 | Br | OCH₃ | 2,5-difluorophenyl | H | (A1); 208°C |
| 160 | B8 | Br | OCH₃ | 2,5-difluorophenyl | H | (A2); 167°C |
| 162 | B7 | Br | OCH₃ | 6-methoxy-2-naphthyl | H | (A); 206°C |
| 163 | B7 | Br | OCH₃ | 6-methoxy-2-naphthyl | H | (B); 206°C |
| 164 | B9 | Br | 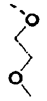 | 3-fluorophenyl | H | (A); 118°C |
| 165 | B9 | Br | 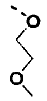 | 3-fluorophenyl | H | (B); oil |
| 167 | B8 | Br | OCH₃ | 2,6-difluorophenyl | H | (B); 180°C |
| 174 | B9 | 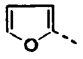 | OCH₃ | 3-fluorophenyl | H | (A); 159°C |
| 175 | B9 | 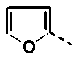 | OCH₃ | 3-fluorophenyl | H | points (B); 196°C |
| 176 | B7 | Br | 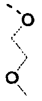 | 1-naphthyl | H | (A); oil |
| 179 | B9 | CN | OCH₃ | 3-fluorophenyl | H | (A); 213°C |
| 180 | B9 | CN | OCH₃ | 3-fluorophenyl | H | (B); 163°C |
| 181 | B9 | Br | OCH₃ | 4-fluorophenyl | H | (A); 198°C |
| 182 | B9 | Br | OCH₃ | 4-fluorophenyl | H | (B); 238°C |
| 183 | B1 | Br | OCH₃ | 3-trifluoromethylphenyl | H | (A); 170°C |
| 188 | B1 | Br | OCH₃ | 1,4-pyrimidin-2-yl | H | (A); 110°C |
| 189 | B1 | Br | OCH₃ | 1,4-pyrimidin-2-yl | H | (B); 145°C |
| 195 | B15 | Br | OCH₃ | 3,4-difluorophenyl | H | (A); 250°C |
| 196 | B15 | Br | OCH₃ | 3,4-difluorophenyl | H | (B); 184°C |
| 201 | B1 | Br | OCH₃ | 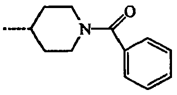 | H | (A); 214°C |
| 202 | B1 | Br | OCH₃ | 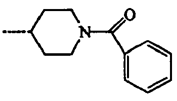 | H | (B); 246°C |
| 203 | B9 | 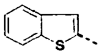 | OCH₃ | 3-fluorophenyl | H | (A); 225°C |
| 204 | B9 | 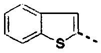 | OCH₃ | 3-fluorophenyl | H | (B); 216°C |
| 205 | B7 | Br | OCH₃ | 1-naphthyl | F | (A); 213°C |
| 206 | B7 | Br | OCH₃ | 1-naphthyl | F | (B); 213 C° |
| 207 | B15 | F | OCH₃ | 3,5-difluorophenyl | H | H(A); 232°C |
| 208 | B15 | F | OCH₃ | 3,5-difluorophenyl | H | (B); 188°C |
| 212 | B7 | 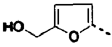 | OCH₃ | 1-naphthyl | H | (B); 220°C |

**Table 2:**

| | | | | | | | |
|---|---|---|---|---|---|---|---|
| 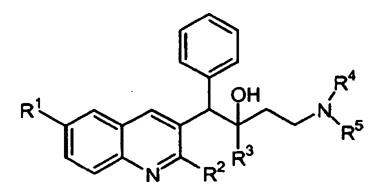 | | | | | | | |

| Comp. nr. | Ex. nr. | R¹ | R² | R³ | R⁴ | R⁵ | Phys.data (salt/melting points) and stereo-chemistry |
|---|---|---|---|---|---|---|---|
| 18 | B1 | Br | OCH₃ | phenyl | CH₂CH₃ | CH₂CH₃ | ethanedioate (2:3); (A); 230°C |
| 19 | B1 | Br | OCH₃ | phenyl | CH₂CH₃ | CH₂CH₃ | ethanedioate (2:3), (B); 150°C |
| 44 | B4 | Br | OCH₃ | phenyl | H | H | (A); 190°C |
| 9 | B4 | Br | OCH₃ | phenyl | H | H | (B); 204°C |
| 141 | B7 | Br | OCH₃ | 2-naphthyl | CH₃ | CH₂CH₃ | (A); 188°C |
| 142 | B7 | Br | OCH₃ | 2-naphthyl | CH₃ | CH₂CH₃ | (B); 202°C |
| 230 | B12 | Br | OCH₃ | 1-naphthyl | CH₃ | benzyl | /oil |
| 147 | B7 | Br | OCH₃ | 1-naphthyl | CH₃ | CH₂CH₃ | (A); 168°C |
| 148 | B7 | Br | OCH₃ | 1-naphthyl | CH₃ | CH₂CH₃ | (B); 212°C |
| 56 | B13 | Br | OCH₃ | 1-naphthyl | CH₃ | H | (A); 204°C |
| 214 | B13 | Br | OCH₃ | 1-naphthyl | CH₃ | H | (B); 225°C |

**Table 3:**

| | | | | |
|---|---|---|---|---|
| 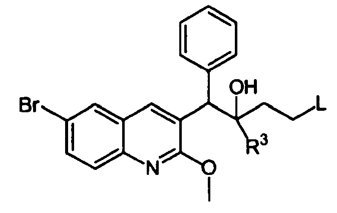 | | | | |

| Comp. nr. | Ex. nr. | R³ | L | Stereochemistry and melting points |
|---|---|---|---|---|
| 47 | B1 | phenyl | 1-piperidinyl | (A); 190°C |
| 48 | B1 | phenyl | 1-piperidinyl | (B); 210°C |
| 128 | B1 | 2-naphthyl | 1-piperidinyl | (A); 254°C |
| 129 | B1 | 2-naphthyl | t-piperidinyt | (B); 212°C |
| 49 | B1 | phenyl | 1-imidazotyl | (A); 216°C |
| 50 | B1 | phenyl | 1-imidazotyl | (B); 230°C |
| 51 | B1 | phenyl | -(4-methyl)piperazinyl | (A); 150°C |
| 52 | B1 | phenyl | 1-(4-methyl)piperazinyl | (B); 230°C |
| 53 | B1 | phenyl | 1-(1,2,4-triazolyl) | (A); 180°C |
| 54 | B1 | phenyl | 1-(1,2,4-triazolyl) | (B); 142°C |
| 55 | B1 | phenyl | thiomorpholinyl | (A); oil |
| 57 | B5 | phenyl | 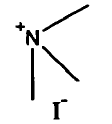 | (A); 244°C |
| 10 | B5 | phenyl | 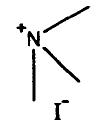 | (B); 198°C |
| 58 | B6 | phenyl | 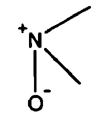 | (A); 208°C |
| 11 | B6 | phenyl | 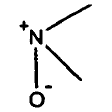 | (B); 208°C |
| 99 | B11 | 1-naphthyl | 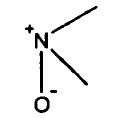 | (A1); 218°C |
| 100 | B6 | 1-naphthyl | 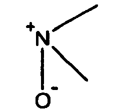 | (A2); 218°C |
| 101 | B6 | 1-naphthyl | 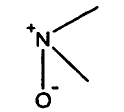 | (B); 175°C |
| 102 | B5 | 1-naphthyl | 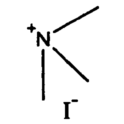 | (A2); 210°C |
| 103 | B5 | 1-naphthyl | 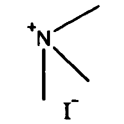 | (B); >250°C |
| 121 | B5 | 1-naphthyl | 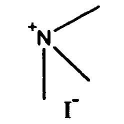 | (A1); 210°C |
| 123 | B1 | phenyl | morpholinyl | (A); 226°C |
| 124 | B1 | phenyl | morpholinyl | (B); 210°C |
| 136 | B7 | 2-naphthyl | 4-methylpyrazinyl | (A); 188°C |
| 137 | B7 | 2-naphthyl | 4-methylpyrazinyl | (B); 232°C |
| 139 | B7 | 2-naphthyl | morphotinyl | (A); 258°C |
| 140 | B7 | 2-naphthyl | morpholinyl | (B); 214°C |
| 144 | B7 | 2-naphthyl | pyrrolidinyl | (A); 238°C |
| 145 | B7 | 1-naphthyl | 1-piperidinyl | (A); 212°C |
| 146 | B7 | 1-naphthyl | 1-piperidinyl | (B); 220°C |
| 149 | B7 | 1-naphthyl | 4-methytpyrazinyl | (B); 232°C |
| 151 | B7 | 3-bromo-1-naphthyl | 4-methytpiperazinyl | (A); 178°C |
| 152 | B7 | 3-bromo-1-naphthyl | 4-methylpiperazinyl | (B); 226°C |
| 153 | B7 | 6-bromo-2-naphthyl | 4-methylpiperazinyl | (A); 208°C |
| 154 | B7 | 6-bromo-2-naphthyl | 4-methylpiperazinyl | (B); 254°C |
| 155 | B7 | 6-bromo-2-naphthyl | 1-piperidinyl | point (A); 224°C |
| 156 | B7 | 1-naphthyl | 4-methylpiperazinyl | (A); 200°C |
| 157 | B7 | 6-bromo-2-naphthyl | 1-pyrrolidinyl | (B); 220°C |
| 158 | B7 | 1-naphthy) | morpholinyl | (B); 272°C |
| 166 | B7 | 6-bromo-2-naphthyl | 1-piperidinyl | (B); 218°C |
| 170 | B7 | 2-naphthyl | 1-pyrrolidinyl | (A); 238°C |
| 171 | B7 | 2-naphthyl | 1-pyrrolidinyl | (B); 218°C |
| 172 | B7 | 1-naphthyl | 1,2,4-triazol-1-yl | /142°C |
| 173 | B7 | 1-naphthyl | 1,2-imidazol-1-yl | (A); 222°C |
| 177 | B7 | 6-bromo-2-naphthyl, | morpholinyl | (A); 242°C |
| 178 | B7 | 6-bromo-2-naphthyl | morpholinyl | (B); 246°C |
| 187 | B7 | 1-naphthyl | 1,2-imidazol-1-yl | (B); 236°C |
| 200 | B7 | 2-naphthyl | 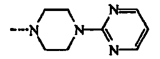 | (A); 254°C |
| 209 | B7 | 2-naphthyl | 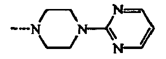 | (B); 198°C |

**Table 4:**

| | | | | | |
|---|---|---|---|---|---|
| 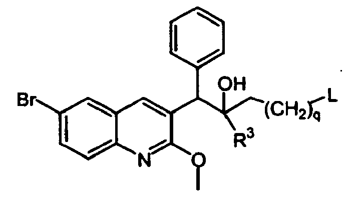 | | | | | |

| Comp. nr. | Ex. nr. | R³ | Q | L | Stereochemistry and melting points |
|---|---|---|---|---|---|
| 61 | B1 | phenyl | 0 | N(CH₃)₂ | (A); 220°C |
| 62 | 1 | phenyl | 0 | N(CH₃)₂ | (B); 194°C |
| 63 | B1 | phenyl | 2 | N(CH₃)₂ | (A); 150°C |
| 64 | 1 | phenyl | 2 | N(CH₃)₂ | (B); 220°C |
| 125 | B7 | 2-naphthyl | 2 | N(CH₃)₂ | (A); 229°C |
| 126 | B7 | 2-naphthyl | 2 | N(CH₃)₂ | (B); 214°C |
| 65 | B1 | phenyl | 3 | N(CH₃)₂ | points (A); 130°C |
| 66 | B1 | phenyl | 3 | N(CH₃)₂ | (B); 170°C |
| 132 | B7 | 2-naphthyl | 2 | pyrrolidinyl | (A); 227°C |
| 133 | B7 | 2-naphthyl | 2 | pyrrolidinyl | (B); 222°C |
| 161 | B7 | 2-naphthyl | 2 | morpholinyl | (B); 234°C |
| 186 | B7 | 1-naphthyl | 2 | N(CH₃)₂ | (A); 187°C |
| 190 | B7 | 2-naphthyl | 3 | N(CH₃)₂ | 170°C (A); 170°C |
| 191 | B7 | 2-naphthyl | 3 | N(CH₃)₂ | (B); 145°C |
| 192 | B7 | 2-naphthyl | 2 | N(CH₂CH₃)₂ | (A); 90°C |
| 193 | B7 | 2-naphthyl | 2 | N(CH₂CH₃)₂ | (B); 202°C |
| 194 | B7 | 1-naphthyl | 2 | pyrrolidinyl | (B); 206°C |
| 197 | B7 | 1-naphthyl | 3 | N(CH₃)₂ | (A); 160°C |
| 198 | B7 | 2-naphthyl | 2 | morpholinyl | (A); 215°C |
| 199 | B7 | 1-naphthyl | 2 | N(CH₂CH₃)₂ | (A); 185°C |
| 210 | B7 | 1-naphthyl | 2 | morpholinyl | (B); 222°C |
| 211 | B7 | 1-naphthyl | 2 | morpholinyl | (A); 184°C |

**Table 5:**

| | | | | | |
|---|---|---|---|---|---|
| 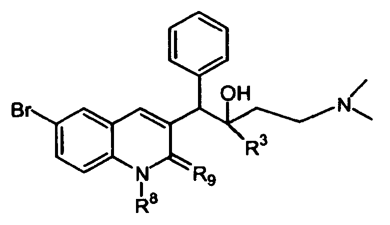 | | | | | |

| Comp. nr. | Ex. nr. | R³ | R⁸ | R⁹ | Stereochemistry and melting points |
|---|---|---|---|---|---|
| 104 | B1 | phenyl | -CH=H-N= | | (A); 170°C |
| 105 | B1 | phenyl | -CH=H-N= | | (B); 150°C |
| 106 | B1 | phenyl | CH₃ | =O | (A); 224°C |
| 107 | B1 | phenyl | CH₃ | =O | (B); 182°C |
| 138 | B7 | 1-naphthyl | H | =O | (A1); >260°C |

**Table 6:**

| | | | | | | | | |
|---|---|---|---|---|---|---|---|---|
| 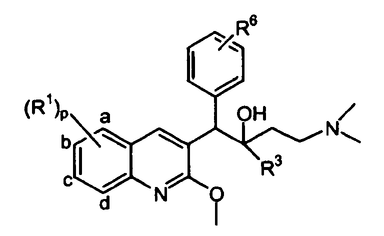 | | | | | | | | |

| Comp. nr. | Ex. nr. | R¹ | | | | R³ | R⁶ | Sterechemistry and melting points |
|---|---|---|---|---|---|---|---|---|
| | | a | b | c | d | | | |
| 215 | B9 | H | Br | CH₃ | H | 3-fluorophenyl | H | (A); 197°C |
| 216 | B9 | H | Br | CH₃ | H | 3-fluorophenyl | H | (B); 158°C |
| 217 | B7 | H | H | Br | H | 1-naphthyl | H | (A); 212°C |
| 218 | B7 | H | H | Br | H | 1-naphthyl | H | (B); 172°C |
| 219 | B9 | H | Br | H | CH₃ | 3-fluorophenyl | H | (A); 220°C |
| 220 | B9 | H | Br | H | CH₃ | 3-fluorophenyl | H | (B); 179°C |
| 221 | B7 | Br | H | H | H | 1-naphthyl | H | (A); 170°C |
| 224 | B7 | Br | H | H | H | 1-naphthyl | H | /205°C |
| 222 | B7 | H | Br | H | H | 1-naphthyl | 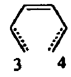 | (A); 155°C |
| 223 | B7 | H | Br | H | H | 1-naphthyl | 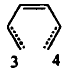 | (B); 205°C |
| 225 | B7 | H | Br | CH₃ | H | 1-naphthyl | H | (A); 238°C |
| 226 | B7 | H | Br | CH₃ | H | 1-naphthyl | H | (B); 208°C |
| 227 | 815 | H | Br | CH₃ | H | 3,5-difluorophenyl | H | (A); 195°C |
| 228 | B15 | H | Br | CH₃ | H | 3,5-difluorophenyl | H | (B); 218°C |
| 229 | B7 | H | CH₃ | CH₃ | H | 1-naphthyl | H | (A); 238°C |

### PHARMACOLOGICAL PART

### A. Study of the effect of final compound 12 in killing dormant Mycobacterium bovis

### Bacterial strains and Culture medium

*Mycobacterium bovis* BCG were obtained from Tibotec Virco (TB0087- (Belgium). *M. bovis* BCG, expressing the luciferase gene on plasmid pSMT1 (a kind gift from Dr. Kris Huygen at Pasteur Institute, Brussles⁸) were cultured in Middlebrook 7H9 medium (Difco, BD271310) with 0.05% Tween-80 (Sigma) in log phase for a period of 3-4 days before start of the experiment.

To prepare growth medium with supplements: dissolve 4.7 g of the Middlebrook powder in 895 ml distilled water and add 5 ml Glycerol, 200 µl Tween 80 and autoclave at 121°C for 15 minutes. Aseptically add 100 ml Middlebrook OADC enrichment to the medium when cooled to 45 °C. Store at 4°C for maximum 1 month. Pre-incubate all media 2 days at 37°C to check for contamination. Add 50 µg/ml hygromycin for strain *M. bovis* BCG expressing the luciferase gene (BCG-pSMT1).

### I. Study with Mycobacterium bovis BCG

### Dormancy assay

500 µl of *Mycobacterium bovis* BCG stock was added to 100 ml Middlebrook 7H9 broth with supplements in a 250 ml sterile Duran bottle with a magnetic stirring rod. Incubation was done on an electric magnetic stirrer for 7 days at 37 °C (500 rpm). 5 ml aliquots of log phase culture (OD₆₀₀ₙₘ =0.5 to 0.8) were transferred into 15 ml screw capped falcon tubes. Various drugs were added to the individual tubes to a final concentration of 10 µg/ml. After the addition of the drugs, all tube were closed loosely and placed inside an anaerobic jar (BBL). Anaerobic gas generation envelopes were used to get anaerobic conditions in the jar and anaerobic strips to monitor the anaerobic conditions. The addition of the individual drugs and the start of the anaerobiosis within the jar was done extremely quickly as previously described⁹. The jar was incubated for 7 days at 37 °C.

### CFU assay

After 7 days of anaerobiosis, the dormant cultures were collected by low speed centrifugation (2000 rpm for 10 minutes). The cells were washed twice with 7H9 medium so as to remove the drugs and resuspended in drug free medium. The CFU of the treated and untreated cultures were determined by plating at day 0, 2, and day 5 to evaluate the bactericidal activity.

### II. Study with M. bovis BCG, expressing the luciferase gene on plasmid pSMT1 Dormancy assay

500 µl of *Mycobacterium bovis* BCG luciferase (pSMT1) stock was added to 100 ml Middlebrook 7H9 broth with supplements in a 250 ml sterile Duran bottle with a magnetic stirring rod. Incubation was done on an electric magnetic stirrer for 7 days at 37°C (500 rpm). 5 ml aliquots of log phase culture (OD₆₀₀ₙₘ =0.5 to 0.8) were transferred into 15 ml screw capped falcon tubes. Various drugs were added to the individual tubes to a final concentration of 10 µg/ml. After addition of the drugs, all tube were closed loosely and placed very quickly inside an anaerobic jar (BBL) as previously described⁹. Anaerobic gas generation envelopes were used to get anaerobic conditions in the jar and anaerobic strips to monitor the anaerobic conditions. The jar was incubated for 7 days at 37 °C.

### Luciferase assay

After 7 days of anaerobiosis, the dormant cultures were collected by low speed centrifugation (2000 rpm for 10 minutes). The cells were washed twice with 7H9 medium so as to remove the drugs and resuspended in drug free medium. After washing, 250 µl of the dormant *M. bovis* BCG luciferase (pSMT1) was added to 5 different microplates (day 0 to day 5). Every sample was diluted in microplates (5-fold dilutions) in medium and incubated again for 37 °C from 0 to 5 days. 40 µl of samples and dilutions were added to 140 µl PB S. 20 µl luciferase substrate (1% n-decyl aldehyde in ethanol) was added. The luminescence was measured for 10 seconds to follow the growth of the viable bacteria on every day from 0 to 5 days (Use Luminoskan Ascent Labsystems with injector).

### Experimental organization:

| **Sample number** | **Strain M.Bovis** | **Sample/compound** | **microgram/ml** |
|---|---|---|---|
| **1-2** | **BCG** | **Control** | |
| **3** | **BCG** | **Metronidazole** | **10** |
| **4** | **BCG** | **Isoniazid** | **10** |
| **5-6** | **BCG** | **Final compound 12** | **10** |
| **7-8** | **BCG** | **Final compound 12** | **1** |
| **9-10** | **BCG** | **Final compound 12** | **0.1** |
| **11-12** | **BCG/pSMT1** | **Control** | |
| **13** | **BCG/pSMT1** | **Metronidazole** | **10** |
| **14** | **BCG/pSMT1** | **Isoniazid** | **10** |
| **15-16** | **BCG/pSMT1** | **Final compound 12** | **10** |
| **17-18** | **BCG/pSMT1** | **Final compound 12** | **1** |
| **19-20** | **BCG/pSMT1** | **Final compound 12** | **0.1** |

### Results and Discussion

An *in vitro* dormancy model of dormancy was developed based on Wayne's method of creating dormant bacteria by oxygen depletion ^{9,10}. In Wayne's model as mycobacteria settle down to the bottom of the flask they generate an oxygen gradient creating anaerobic conditions at the bottom of the flask. This transition to low oxygen concentrations causes mycobacteria to become dormant and that leads to upregulated expression of several genes including isocitrate lyase and glycine dehydrogenase⁷. These enzymes are responsible for production of energy in absence of oxygen and the terminal electron acceptors are nitrate, sulfates etc as compared to molecular oxygen in case of aerobic respiration. The energy of reduced substrates generates a electron chemical gradient.

In this experiment, an adaptation of Wayne's model was used in the experimental set up involving the use of gaspak anaerobic jars in which oxygen is depleted in the chamber by means of a chemical reaction⁹. Gaspak jars are fitted with a lid containing a catalyst. A Gaspak foil envelope containing substances that generate hydrogen and CO₂ is placed in the jar with the bacterial cultures. The envelope is opened, and 10 ml of tap water is pipetted into it. When the jar is closed (the lid is clamped down tightly), the hydrogen given off combines with oxygen, through the mediation of the catalyst, to form water. This leads to the gradual depletion of the oxygen present in the chamber and as such creates the oxygen gradient. Furthermore, an indicator strip in the jar contains methylene blue, which turns colourless in the absence of oxygen. The colour change in the indicator strip signifies that the proper atmospheric condition has been achieved.

For rapid analysis of the effect of the compound on the dormant bacteria, *M. bovis* BCG transformed with the luciferase construct was used. *M. bovis* BCG has been used in earlier experiments as a surrogate to mimic dormancy in mycobacteria in general and *M. tuberculosis* in particular^{11,12}. Luciferase reporter strains have been used quite often to access the viability of the bacteria ^{13,14}. The *M. bovis* BCG is transformed with the reporter plasmid pSMT1, which is a shuttle vector containing the origin of replication of *E. coli* and mycobacteria⁸. The luminescent genes from *Vibro harveyi* (lux A and B) are under control of BCG hsp60 promoter and produce light in presence of ATP or Flavin mononucleotide (FMNH₂). Dead cells are not able to produce these cofactors, thus corresponding to decline in luminescence.

The activity of final compound 12 in this dormancy assay was analysed as well as the activity of other drugs including metronidazole and Isoniazid. Dormant bacteria are not killed by Isoniazid and to some extent are also resistant to rifampicin but are susceptible to killing by metronidazole, an antibiotic for anaerobic pathogens ^{15,16}. Isoniazid acts as an early bactericidal agent and its activity is limited to killing of replicating bacilli but does not have a significant sterilizing activity on dormant bacilli 17

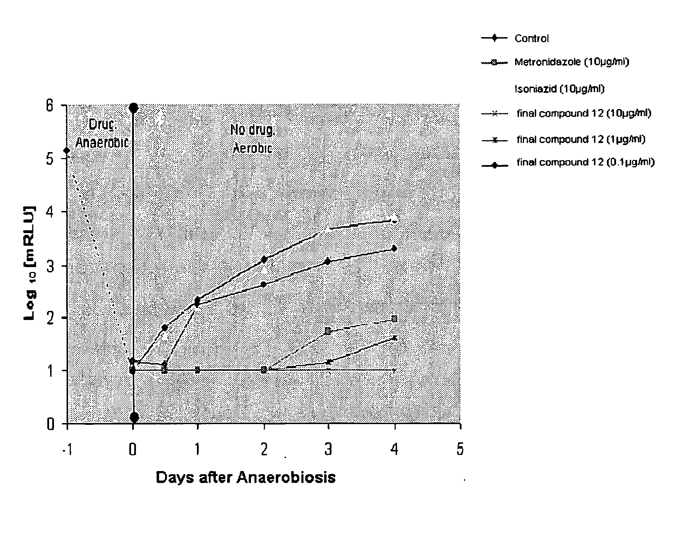

After 7 days of anaerobiosis, the bacteria were suspended in drug free medium for 5 days and the effect of different compounds on bacterial viability was assayed by Luciferase counts. As shown in Figure 1, Isoniazid had no effect on these dormant bacteria and these bacteria had almost similar growth characteristics as compared to control, demonstrating the dormant or non-replicating status of the cultured bacilli. In contrast, metronidazole was clearly effective in killing the dormant bacilli over a period of time with reduction of 2 log₁₀ as compared to control. Final compound 12 affects the survival of the dormant bacteria in concentration dependent manner. At 10 µg/ml concentration of final compound 12 there was approximately 4-log₁₀ reduction in bacterial survival as compared to untreated control. At 0.1 and 1 µg/ml of the compound the corresponding killing of dormant bacteria was about 0.5 log₁₀ and 2 log₁₀ respectively.

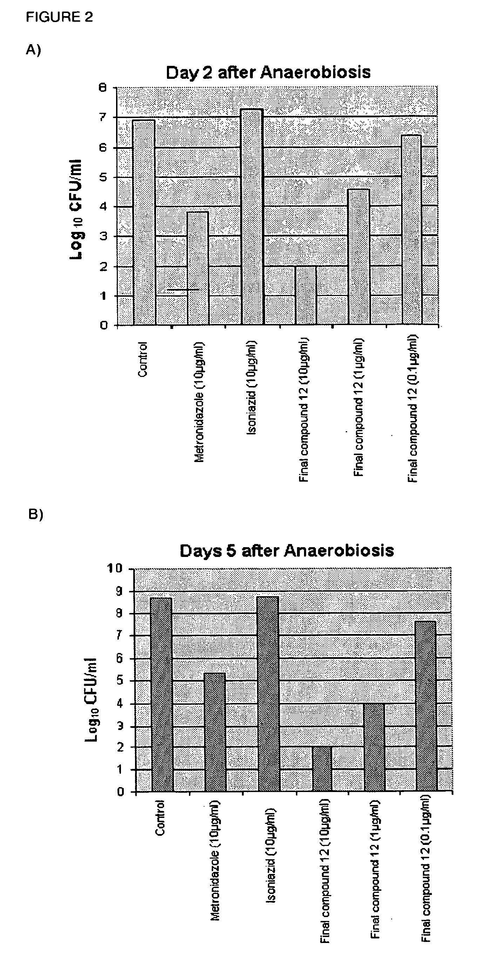

To correlate the effects of final compound 12 on bacteria killing in terms relative luminescence units (RLU/ml) versus colony forming units (CFU/ ml), bacterial counts were also measured on 7H10 plates. A similar ratio of RLU units with the CFU counts was observed after plating the day 2 and day 4 samples on 7H10 plates. The reduction in CFU counts compared with that of untreated control showed that final compound 12 at 10 µg/ml, 1 µg/ml and 0.1 µg/ml reduced the viability by approximately 4, 2.3, and 0.5 log₁₀ at day 2 and about 6, 4.7 and 1.1 log₁₀ at day 5 respectively. Fig 2 (A and B) reports CFU data. A close correlation was observed between luminescence and the CFU during various stages of the experiment. Interestingly there was marked reduction in RLUs at time point 0 as compared to CFU counts, primarily because ATP concentration within these cells is very low, which has been shown to be the characteristics of the metabolic state of the dormant bacilli⁸ .

The activity of final compound 12 on dormant (non-multiplying) mycobacteria is an extremely important finding, as it will help in the fight against tuberculosis by eradicating the disease in individuals who are at risk of developing TB.

### B. Study of the effect of present compounds in killing dormant Mycobacterium tuberculosis according to the Wayne dormancy model*

### Bacterial strain and Culture medium

*Mycobacterium tuberculosis* (H37RV) was cultured in Middlebrook medium with 0.05% Tween.

To prepare Middlebrook 7H9 Broth (1X) (BD 271310) with supplements :dissolve 4.7 g of the Middlebrook powder in 895 ml distilled water and add 5 ml Glycerol, 200 µl Tween 80 and autoclave at 121 °C for 15 minutes. Aseptically add 100 ml Middlebrook OADC Enrichment (BD 211886) to the medium when cooled to 45 °C. Store at 4 °C for maximum 1 month. Pre-incubate all media 2 days at 37 °C to check for contamination.

* Wayne L.G. et al.; Infection and Immunity 64 (6), 2062-2069 (1996)

### Study with Mycobacterium tuberculosis (H37RV)

### Dormancy assay

1000 µl of *Mycobacterium tuberculosis* stock (previous culture) was added to 100 ml Middlebrook 7H9 broth with supplements in a 250 ml sterile Duran bottle with a magnetic stirring rod. Incubation was done on an electric magnetic stirrer for 7 days at 37 °C (500 rpm). 17 ml aliquots of log phase culture (calculated OD₆₀₀ₙₘ = 0.01) were transferred into 25 ml tubes. The tubes were tightly closed with caps with rubber septa and incubated on a magnetic stirring plate to create anaerobiosis by oxygen depletion. Stirring in the tubes was achieved with 8 mm teflon stirring bar. The tubes were incubated for 22 days at 37 °C in an incubator on a magnetic stirring plate (120 rpm) until anaerobiosis (methylene blue (1.5 mg/liter) is turned to colourless). After 14 days various drugs (final concentration of 100, 10, 1 and 0.1 µg/ml) were added to the individual tubes. Metronidazole was added as control to kill the dormant bacteria (added at start). Isoniazid was added as control to show that it does not have any effect on growth and viability of dormant bacteria.

### CFU assay

After 22 days, the cultures were collected by low speed centrifugation (2000 rpm for 10 minutes). The cells were washed twice with drug free medium and the cells were resuspended in drug-free medium and incubated. The reduction in CFU compared to untreated control cultures, was determined by plating after anaerobiosis to evaluate the bactericidal activity. -

### Experimental organization

| **Sample number** | **Sample / Compound** | **µg / ml** |
|---|---|---|
| 1 - 2 | Control | - |
| 3 - 4 | Metronidazole | 100 |
| 5 - 6 | Isoniazid | 10 |
| 7 - 8 | Moxifloxacin | 10 |
| 9 - 10 | | 1 |
| 11 - 12 | Final compound 12 | 10 |
| 13 - 14 | | 1 |
| 15 - 16 | Rifampicin | 10 |
| 17 - 18 | | 1 |

### Results and Discussion:

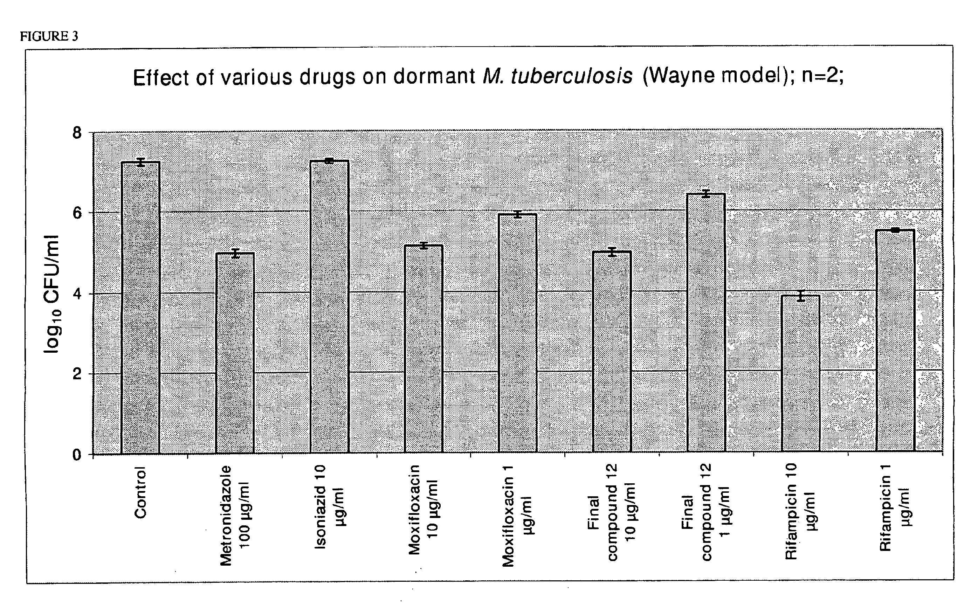

The effect of the final compound 12 on dormant bacteria is demonstrated (see Fig. 3) using the Wayne dormancy model. As already indicated above, it is an *in vitro* oxygen depletion model, which triggers a dormancy response in the bacteria¹⁸⁻²³. In Wayne model, cultures of the bacterium are subjected to the gradual oxygen depletion by incubation in stirred sealed tubes. With slow shift of the aerobic growing bacteria to anaerobic conditions, the culture is more capable to adapt and survive the anaerobiosis by shifting down to a state of anaerobic persistence. Wayne model is a well-characterized *in vitro* model for dormancy.

At 10µg/ml concentration of final compound 12, more than 2 log₁₀ reduction of the dormant bacteria was observed as also seen in case of moxifloxacin and rifampin. At 1µg/ml concentration, 1.41 log₁₀ reduction was observed for compound 12. Compounds 71, 75, 172 and 125 were also tested in the same test At 10µg/ml concentration, more than 2 log₁₀ reduction of the dormant bacteria was observed for compound 71; 1.14 log₁₀ reduction was observed for compound 75; 0.98 log₁₀ reduction was observed for compound 172; 0.23 log₁₀ reduction was observed for compound 125. At 1µg/ml concentration, 1.55 log₁₀ reduction was observed for compound 71; 0.87 log₁₀ reduction was observed for compound 75; 0.29 log₁₀ reduction was observed for compound 172.

Isoniazid did not have any effect on dormant bacteria while the control compound, metronidazole showed good efficacy.

### Reference List

1. Corbett,E.L. et al. The growing burden of tuberculosis: global trends and interactions with the HIV epidemic. Arch. Intern. Med 163, 1009-1021 (2003).
2. Dye,C., Scheele,S., Dolin,P., Pathania,V. & Raviglione,M.C. Consensus statement. Global burden of tuberculosis: estimated incidence, prevalence, and mortality by country. WHO Global Surveillance and Monitoring Project. JAMA 282, 677-686 (1999).
3. Targeted tuberculin testing and treatment of latent tuberculosis infection. This official statement of the American Thoracic Society was adopted by the ATS Board of Directors, July 1999. This is a Joint Statement of the American Thoracic Society (ATS) and the Centers for Disease Control and Prevention (CDC). This statement was endorsed by the Council of the Infectious Diseases Society of America. (IDSA), September 1999, and the sections of this statement. Am J Respir. Crit Care Med 161, S221-S247 (2000).
4. Halsey,N.A. et al. Randomised trial of isoniazid versus rifampicin and pyrazinamide for prevention of tuberculosis in HIV-1 infection. Lancet 351, 786-792 (1998).
5. Mitchison,D.A. & Coates, A.R. Predictive in vitro models of the sterilizing activity of anti-tuberculosis drugs. Curr. Pharm. Des 10, 3285-3295 (2004).
6. Karakousis,P.C. et al. Dormancy phenotype displayed by extracellular Mycobacterium tuberculosis within artificial granulomas in mice. J Exp. Med 200, 647-657 (2004).
7. Gomez,J.E. & McKinney,J.D. M. tuberculosis persistence, latency, and drug tolerance. Tuberculosis. (Edinb.) 84, 29-44 (2004).
8. Snewin,V.A. et al. Assessment of immunity to mycobacterial infection with luciferase reporter constructs. Infect. Immun. 67, 4586-4593 (1999).
9. Stover, C.K. et al. A small-molecule nitroimidazopyran drug candidate for the treatment of tuberculosis. Nature 405, 962-966 (2000).
10. Wayne,L.G. Synchronized replication of Mycobacterium tuberculosis. Infect. Immun. 17, 528-530 (1977).
11. Boon,C. & Dick,T. Mycobacterium bovis BCG response regulator essential for hypoxic dormancy. J Bacteriol. 184, 6760-6767 (2002).
12. Hutter,B. & Dick,T. Up-regulation of narX, encoding a putative 'fused nitrate reductase' in anaerobic dormant Mycobacterium bovis BCG. FEMS Microbiol. Lett. 178, 63-69 (1999).
13. Andrew,P.W. & Roberts,I.S. Construction of a bioluminescent mycobacterium and its use for assay ofantimycobacterial agents. J Clin Microbiol. 31, 2251-2254 (1993).
14. Hickey,M.J. et al. Luciferase in vivo expression technology: use of recombinant mycobacterial reporter strains to evaluate antimycobacterial activity in mice. Antimicrob. Agents Chemother. 40, 400-407 (1996).
15. Wayne,L.G. & Sramek,H.A. Metronidazole is bactericidal to dormant cells of Mycobacterium tuberculosis. Antimicrob. Agents Chemother. 38, 2054-2058 (1994).
16. Wayne,L.G. Dormancy of Mycobacterium tuberculosis and latency of disease. Eur. J Clin Microbiol. Infect. Dis 13, 908-914 (1994).
17. Lalande,V., Truffot-Pernot,C., Paccaly-Moulin,A., Grosset,J. & Ji,B. Powerful bactericidal activity of sparfloxacin (AT-4140) against Mycobacterium tuberculosis in mice. Antimicrob. Agents Chemother. 37, 407-413 (1993).
18. Zhang,Y. Persistent and dormant tubercle bacilli and latent tuberculosis. Front Biosci. 9, 1136-1156 (2004).
19. Boon,C. & Dick,T. Mycobacterium bovis BCG response regulator essential for hypoxic dormancy. J Bacteriol. 184, 6760-6767 (2002).
20. Peh,H.L., Toh,A., Murugasu-Oei,B. & Dick,T. In vitro activities of mitomycin C against growing and hypoxic dormant tubercle bacilli. Antimicrob. Agents Chemother. 45, 2403-2404 (2001).
21. Hutter,B. & Dick,T. Increased alanine dehydrogenase activity during dormancy in Mycobacterium smegmatis. FEMS Microbiol. Lett. 167, 7-11 (1998).
22. Wayne,L.G. & Hayes,L.G. An in vitro model for sequential study of shiftdown of Mycobacterium tuberculosis through two stages of nonreplicating persistence. Infect. Immun. 64, 2062-2069 (1996).
23. Antia,R., Koella,J.C. & Perrot,V. Models of the within-host dynamics of persistent mycobacterial infections. Proc. Biol. Sci. 263, 257-263 (1996).

## Claims

1. Use of a compound of formula (Ia) or (Ib) for the manufacture of a medicament for the treatment of latent tuberculosis, wherein the compound of formula (Ia) or (Ib) is 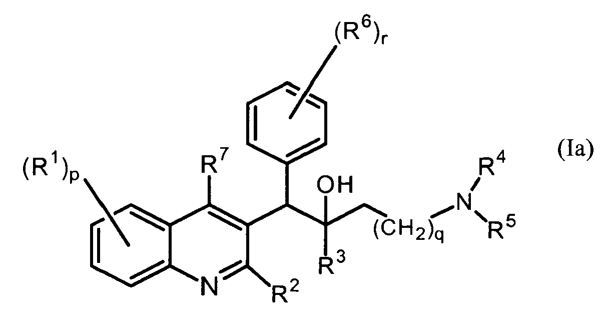 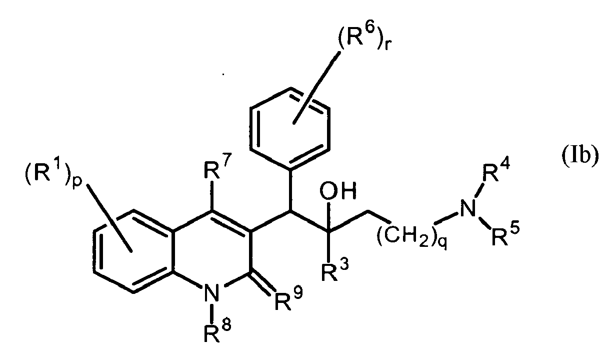 a pharmaceutically acceptable acid or base addition salt thereof, a quaternary amine thereof, a *N*-oxide thereof, a tautomeric form thereof or a stereochemically isomeric form thereof wherein
R¹ is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
p is an integer equal to 1, 2, 3 or 4 ;
R² is hydrogen, hydroxy, mercapto, alkyloxy, alkyloxyalkyloxy, alkylthio, mono or di(alkyl)amino or a radical of formula 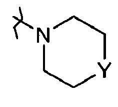 wherein Y is CH₂, O, S, NH or *N*-alkyl ;
R³ is alkyl, Ar, Ar-alkyl, Het or Het-alkyl;
q is an integer equal to zero, 1, 2, 3 or 4 ;
R⁴ and R⁵ each independently are hydrogen, alkyl or benzyl; or
R⁴ and R⁵ together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, pyridazinyl, pyrimidinyl, pyrazinyl, triazinyl, morpholinyl and thiomorpholinyl, each of said ring systems optionally substituted with alkyl, halo, haloalkyl, hydroxy, alkyloxy, amino, mono- or dialkylamino, alkylthio, alkyloxyalkyl, alkylthioalkyl and pyrimidinyl ;
R⁶ is hydrogen, halo, haloalkyl, hydroxy, Ar, alkyl, alkyloxy, alkylthio, alkyloxyalkyl, alkylthioalkyl, Ar-alkyl or di(Ar)alkyl ; or
two vicinal R⁶ radicals may be taken together to form a bivalent radical of formula -CH=CH-CH=CH- ;
r is an integer equal to 1, 2, 3, 4 or 5 ;
R⁷ is hydrogen, alkyl, Ar or Het ;
R⁸ is hydrogen or alkyl ;
R⁹ is oxo ; or
R⁸ and R⁹ together form the radical =N-CH=CH-;
alkyl is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo, hydroxy, alkyloxy or oxo ;
Ar is a homocycle selected from the group of phenyl, naphthyl, acenaphthyl, tetrahydronaphthyl, each homocycle optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
Het is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3- dihydrobenzo[1,4]dioxinyl or benzo[1,3]dioxolyl ; each monocyclic and bicyclic heterocycle may optionally be substituted with 1, 2 or 3 substituents selected from the group of halo, hydroxy, alkyl, alkyloxy or Ar-carbonyl;
halo is a substituent selected from the group of fluoro, chloro, bromo and iodo; and
haloalkyl is a straight or branched saturated hydrocarbon radical having from 1 to 6 carbon atoms or a cyclic saturated hydrocarbon radical having from 3 to 6 carbon atoms, wherein one or more carbon atoms are substituted with one or more halo-atoms.

2. Use according to claim 1 wherein
R¹ is hydrogen, halo, cyano, Ar, Het, alkyl, and alkyloxy ;
p is an integer equal to 1 or 2;
R² is hydrogen, hydroxy, alkyloxy, alkyloxyalkyloxy, alkylthio or a radical of formula 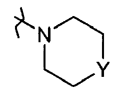 wherein Y is O;
R³ is alkyl, Ar, Ar-alkyl or Het ;
q is an integer equal to zero, 1, 2, or 3 ;
R⁴ and R⁵ each independently are hydrogen, alkyl or benzyl; or
R⁴ and R⁵ together and including the N to which they are attached may form a radical selected from the group of pyrrolidinyl, imidazolyl, triazolyl, piperidinyl, piperazinyl, pyrazinyl, morpholinyl and thiomorpholinyl, each ring system optionally substituted with alkyl or pyrimidinyl ;
R⁶ is hydrogen, halo or alkyl ; or
two vicinal R⁶ radicals may be taken together to form a bivalent radical of formula -CH=CH-CH=CH- ;
r is an integer equal to 1 ;
R⁷ is hydrogen ;
R⁸ is hydrogen or alkyl ;
R⁹ is oxo ; or
R⁸ and R⁹ together form the radical =N-CH=CH-;
alkyl is a straight or branched saturated hydrocarbon radical having from 1 to 6 carbon atoms ; or is a cyclic saturated hydrocarbon radical having from 3 to 6 carbon atoms ; or is a a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo or hydroxy ;
Ar is a homocycle selected from the group of phenyl, naphthyl, acenaphthyl, tetrahydronaphthyl, each homocycle optionally substituted with 1, 2 or 3 substituents, each substituent independently selected from the group of halo, haloalkyl, cyano, alkyloxy and morpholinyl ;
Het is a monocyclic heterocycle selected from the group of *N*-phenoxypiperidinyl, piperidinyl, furanyl, thienyl, pyridinyl, pyrimidinyl ; or a bicyclic heterocycle selected from the group ofbenzothienyl, 2,3-dihydrobenzo[1,4]dioxinyl or benzo[1,3]dioxolyl; each monocyclic and bicyclic heterocycle may optionally be substituted with 1, 2 or 3 alkyl or Ar-carbonyl substituents ; and
halo is a substituent selected from the group of fluoro, chloro and bromo.

3. Use according to claim 1 or 2 wherein in formula (Ia) or (Ib) R¹ is hydrogen, halo, Ar, alkyl or alkyloxy.

4. Use according to claim 3 wherein R¹ is halo.

5. Use according to any one of the preceding claims wherein in formula (Ia) or (Ib) p is equal to 1.

6. Use according to any one of the preceding claims wherein in formula (Ia) or (Ib) R² is hydrogen, alkyloxy or alkylthio.

7. Use according to claim 6 wherein R² is alkyloxy.

8. Use according to any one of the preceding claims wherein in formula (Ia) or (Ib) R³ is naphthyl, phenyl or thienyl, each optionally substituted with 1 or 2 substituents selected from the group of halo and haloalkyl.

9. Use according to claim 8 wherein R³ is naphthyl.

10. Use according to any one of the preceding claims wherein in formula (Ia) or (Ib) q is equal to 1.

11. Use according to any one of the preceding claims wherein in formula (Ia) or (Ib) R⁴ and R⁵ each independently are hydrogen or alkyl or R⁴ and R⁵ together and including the N to which they are attached form a radical selected from the group of imidazolyl, triazolyl, piperidinyl, piperazinyl and thiomorpholinyl.

12. Use according to claim 11 wherein in formula (Ia) or (Ib) R⁴ and R⁵ each independently are hydrogen or alkyl.

13. Use according to claim 12 wherein R⁴ and R⁵ are C₁₋₄alkyl.

14. Use according to any one of the preceding claims wherein in formula (Ia) or (Ib) R⁶ is hydrogen, alkyl or halo.

15. Use according to claim 14 wherein R⁶ is hydrogen.

16. Use according to any one of the preceding claims wherein in formula (Ia) or (Ib) r is equal to 1.

17. Use according to any one of the preceding claims wherein in formula (Ia) or (Ib) R⁷ is hydrogen.

18. Use according to claim 1 wherein in formula (Ia) or (Ib) R¹ is hydrogen, halo, Ar, alkyl or alkyloxy; p = 1; R² is hydrogen, alkyloxy or alkylthio; R³ is naphthyl, phenyl or thienyl, each optionally substituted with 1 or 2 substituents selected from the group of halo and haloalkyl; q = 0, 1, 2 or 3; R⁴ and R⁵ each independently are hydrogen or alkyl or R⁴ and R⁵ together and including the N to which they are attached form a radical selected from the group of imidazolyl, triazolyl, piperidinyl, piperazinyl and thiomorpholinyl; R⁶ is hydrogen, alkyl or halo; r is equal to 1 and R⁷ is hydrogen.

19. Use according to any one of the preceding claims wherein alkyl represents C₁₋₆alkyl.

20. Use according to any one of the preceding claims wherein haloalkyl represents polyhaloC₁₋₆alkyl.

21. Use according to any one of the preceding claims wherein the compound is a compound according to formula (Ia).

22. Use according to claim 1, **characterized in that** the compound is selected from the group consisting of:
ο 1-(6-bromo-2-methoxy-quinolin-3-yl)-2-(3,5-difluoro-phenyl)-4-dimethylamino-1-phenyl-butan-2-ol ;
o 1-(6-bromo-2-methoxy-quinolin-3-yl)-4-dimethylamino-2-naphthalen-1-yl-1-phenyl-butan-2-ol ;
o 1-(6-bromo-2-methoxy-quinolin-3-yl)-2-(2,5-difluoro-phenyl)-4-dimethylamino-1-phenyl-butan-2-ol ;
o 1-(6-bromo-2-methoxy-quinolin-3-yl)-2-(2,3-difluoro-phenyl)-4-dimethylamino-1-phenyl-butan-2-ol ;
o 1-(6-bromo-2-methoxy-quinolin-3-yl)-4-dimethylamino-2-(2-fluoro-phenyl)-1-phenyl-butan-2-ol ;
o 1-(6-bromo-2-methoxy-quinolin-3-yl)-4-dimethylamino-2-naphthalen-1-yl-1-*p-*tolyl-butan-2-ol;
o 1-(6-bromo-2-methoxy-quinolin-3-yl)-4-methylamino-2-naphthalen-1-yl-1-phenyl-butan-2-ol;
o 1-(6-bromo-2-methoxy-quinolin-3-yl)-4-dimethylamino-2-(3-fluoro-phenyl)-1-phenyl-butan-2-ol; and
o 1-(6-bromo-2-methoxy-quinolin-3-yl)-4-dimethylamino-2-phenyl-1-phenyl-butan-2-ol;
a pharmaceutically acceptable acid or base addition salt thereof, a *N*-oxide thereof or a stereochemically isomeric form thereof.

23. Use according to claim 1 wherein the compound is selected from the group consisting of
o 1-(6-bromo-2-methoxy-quinolin-3-yl)-2-(2,3-difluoro-phenyl)-4-dimethylamino-1-phenyl-butan-2-ol;
o 1-(6-bromo-2-methoxy-quinolin-3-yl)-4-dimethylamino-2-naphthalen-1-yl-1-phenyl-butan-2-ol;
a pharmaceutically acceptable acid or base addition salt thereof, a *N*-oxide thereof or a stereochemically isomeric form thereof.

24. Use according to claim 1 wherein the compound is 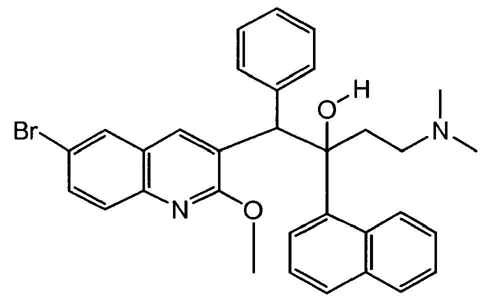 a pharmaceutically acceptable acid or base addition salt thereof, a *N*-oxide thereof, or a stereochemically isomeric form thereof.

25. Use according to claim 24 wherein the compound is 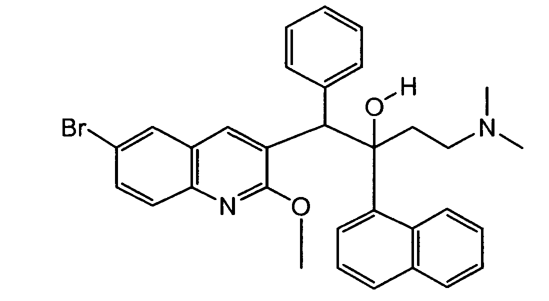 or a pharmaceutically acceptable acid addition salt thereof.

26. Use according to claim 24 wherein the compound is 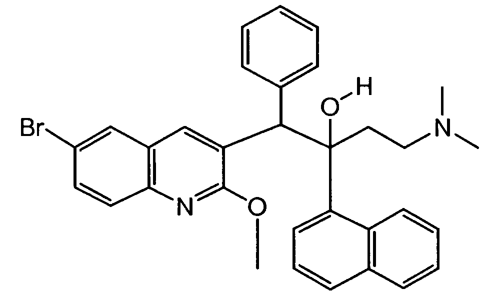 or a stereochemically isomeric form thereof.

27. Use according to claim 24 wherein the compound is 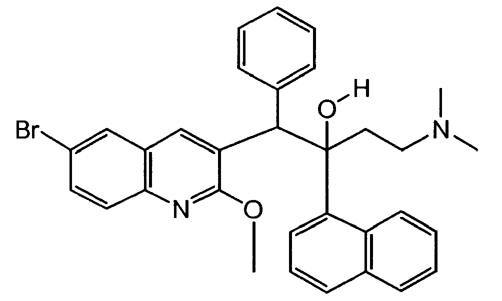 or a *N*-oxide form thereof.

28. Use according to claim 24 wherein the compound is 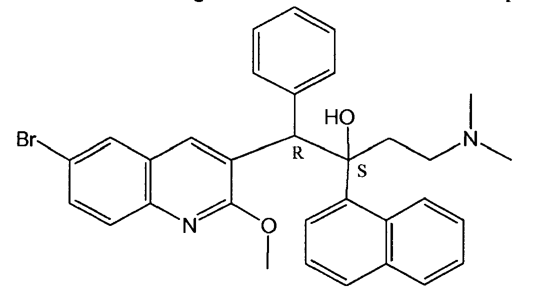 or a pharmaceutically acceptable acid addition salt thereof

29. Use according to claim 28 wherein the compound is

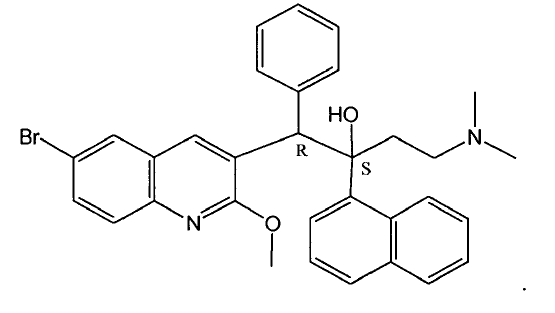

## Patentansprüche

1. Verwendung einer Verbindung der Formel (Ia) oder (Ib) zur Herstellung eines Arzneimittels zur Behandlung von latenter Tuberkulose, wobei es sich bei der Verbindung der Formel (Ia) oder (Ib) um 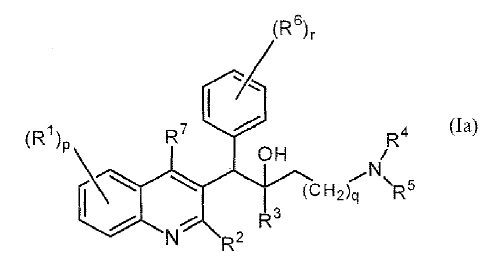 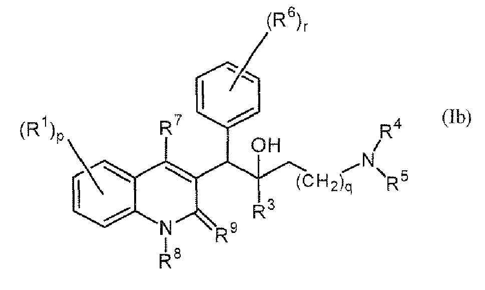 ein pharmazeutisch annehmbares Säure- oder Basenadditionssalz davon, ein quartäres Amin davon, ein *N*-Oxid davon, eine tautomere Form davon oder eine stereochemisch isomere Form davon handelt, wobei
R¹ für Wasserstoff, Halogen, Halogenalkyl, Cyano, Hydroxy, Ar, Het, Alkyl, Alkyloxy, Alkylthio, Alkyloxyalkyl, Alkylthioalkyl, Ar-Alkyl oder Di(Ar)alkyl steht;
p für eine ganze Zahl mit einem Wert von 1, 2, 3 oder 4 steht;
R² für Wasserstoff, Hydroxy, Mercapto, Alkyloxy, Alkyloxyalkyloxy, Alkylthio, Mono- oder Di(alkyl)amino oder einen Rest der Formel 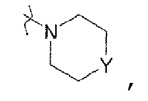 worin Y CH₂, O, S, NH oder *N*-Alkyl bedeutet, steht;
R³ für Alkyl, Ar, Ar-Alkyl, Het oder Het-Alkyl steht;
q für eine ganze Zahl mit einem Wert von Null, 1, 2, 3 oder 4 steht;
R⁴ und R⁵ jeweils unabhängig für Wasserstoff, Alkyl oder Benzyl stehen; oder
R⁴ und R⁵ gemeinsam und einschließlich des N, an das sie gebunden sind, einen Rest aus der Gruppe Pyrrolidinyl, 2-Pyrrolinyl, 3- Pyrrolinyl, Pyrrolyl, Imidazolidinyl, Pyrazolidinyl, 2-Imidazolinyl, 2-Pyrazolinyl, Imidazolyl, Pyrazolyl, Triazolyl, Piperidinyl, Pyridinyl, Piperazinyl, Pyridazinyl, Pyrimidinyl, Pyrazinyl, Triazinyl, Morpholinyl und Thiomorpholinyl bilden können, wobei jedes der Ringsysteme gegebenenfalls durch Alkyl, Halogen, Halogenalkyl, Hydroxy, Alkyloxy, Amino, Mono- oder Dialkylamino, Alkylthio, Alkyloxyalkyl, Alkylthioalkyl und Pyrimidinyl substituiert ist;
R⁶ für Wasserstoff, Halogen, Halogenalkyl, Hydroxy, Ar, Alkyl, Alkyloxy, Alkylthio, Alkyloxyalkyl, Alkylthioalkyl, Ar-Alkyl oder Di(Ar)alkyl steht; oder
zwei vicinale Reste R⁶ zusammengenommen einen zweiwertigen Rest der Formel -CH=CH-CH=CH- bilden können;
r für eine ganze Zahl mit einem Wert von 1, 2, 3, 4 oder 5 steht;
R⁷ für Wasserstoff, Alkyl, Ar oder Het steht;
R⁸ für Wasserstoff oder Alkyl steht;
R⁹ für Oxo steht; oder
R⁸ und R⁹ gemeinsam den Rest =N-CH=CH- bilden;
Alkyl für einen geradkettigen oder verzweigten gesättigten Kohlenwasserstoffrest mit 1 bis 6 Kohlenstoffatomen oder einen cyclischen gesät- tigten Kohlenwasserstoffrest mit 3 bis 6 Koh- lenstoffatomen oder einen cyclischen gesättig- ten Kohlenwasserstoffrest mit 3 bis 6 Kohlen- stoffatomen, der an einen geradkettigen oder verzweigten gesättigten Kohlenwasserstoffrest mit 1 bis 6 Kohlenstoffatomen gebunden ist, steht; wobei jedes Kohlenstoffatom gegebenen- falls durch Halogen, Hydroxy, Alkyloxy oder Oxo substituiert sein kann;
Ar für einen Homocyclus aus der Gruppe Phenyl, Naphthyl, Acenaphthyl, Tetrahydronaphthyl steht, wobei jeder Homocyclus gegebenenfalls durch 1, 2 oder 3 Substituenten substituiert ist, wobei jeder Substituent unabhängig aus der Gruppe Hydroxy, Halogen, Cyano, Nitro, Amino, Mono- oder Dialkylamino, Alkyl, Halogenalkyl, Alkyloxy, Halogenalkyloxy, Carboxyl, Alkyloxy- carbonyl, Aminocarbonyl, Morpholinyl und Mono- oder Dialkylaminocarbonyl ausgewählt ist;
Het für einen monocyclischen Heterocyclus aus der Gruppe *N*-Phenoxypiperidinyl, Piperidinyl, Pyrrolyl, Pyrazolyl, Imidazolyl, Furanyl, Thienyl, Oxazolyl, Isoxazolyl, Thiazolyl, Isothiazolyl, Pyridinyl, Pyrimidinyl, Pyrazinyl und Pyridazinyl oder einen bicyclischen Heterocyclus aus der Gruppe Chinolinyl, Chinoxalinyl, Indolyl, Benzimidazolyl, Benzoxazolyl, Benzisoxazolyl, Benzothiazolyl, Benzisothiazolyl, Benzofuranyl, Benzothienyl, 2,3-Dihydrobenzo[1,4]dioxinyl oder Benzo[1,3]- dioxolyl steht; wobei jeder monocyclische und bicyclische Heterocyclus gegebenenfalls durch 1, 2 oder 3 Substituenten aus der Gruppe Halogen, Hydroxy, Alkyl, Alkyloxy oder Ar- Carbonyl substituiert sein kann;
Halogen für einen Substituenten, aus der Gruppe Fluor, Chlor, Brom und Iod steht; und
Halogenalkyl für einen geradkettigen oder verzweigten gesättigten Kohlenwasserstoffrest mit 1 bis 6 Kohlenstoffatomen oder einen cyclischen gesättigten Kohlenwasserstoffrest mit 3 bis 6 Kohlenstoffatomen steht, wobei ein oder mehrere Kohlenstoffatome durch ein oder mehrere Halogenatome substituiert sind.

2. Verwendung nach Anspruch 1, wobei
R¹ für Wasserstoff, Halogen, Cyano, Ar, Het, Alkyl und Alkyloxy steht;
p für eine ganze Zahl mit einem Wert von 1 oder 2 steht;
R² für Wasserstoff, Hydroxy, Alkyloxy, Alkyloxy- alkyloxy, Alkylthio oder einen Rest der Formel 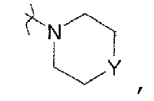 in der Y O bedeutet, steht;
R³ für Alkyl, Ar, Ar-Alkyl oder Het steht;
q für eine ganze Zahl mit einem Wert von Null, 1, 2 oder 3 steht;
R⁴ und R⁵ jeweils unabhängig für Wasserstoff, Alkyl oder Benzyl stehen; oder
R⁴ und R⁵ gemeinsam und einschließlich des N, an das sie gebunden sind, einen Rest aus der Gruppe Pyrrolidinyl, Imidazolyl, Triazolyl, Piperidinyl, Piperazinyl, Pyrazinyl, Morpholinyl und Thiomorpholinyl bilden können, wobei jedes Ringsystem gegebenenfalls durch Alkyl oder Pyrimidinyl substituiert ist;
R⁶ für Wasserstoff, Halogen oder Alkyl steht; oder
zwei vicinale Reste R⁶ zusammengenommen einen zweiwertigen Rest der Formel -CH=CH-CH=CH- bilden können;
r für eine ganze Zahl mit einem Wert von 1 steht;
R⁷ für Wasserstoff steht;
R⁸ für Wasserstoff oder Alkyl steht;
R⁹ für Oxo steht; oder
R⁸ und R⁹ gemeinsam den Rest =N-CH=CH- bilden;
Alkyl für einen geradkettigen oder verzweigten gesättigten Kohlenwasserstoffrest mit 1 bis 6 Kohlenstoffatomen oder einen cyclischen gesät- tigten Kohlenwasserstoffrest mit 3 bis 6 Koh- lenstoffatomen oder einen cyclischen gesättig- ten Kohlenwasserstoffrest mit 3 bis 6 Kohlen- stoffatomen, der an einen geradkettigen oder verzweigten gesättigten Kohlenwasserstoffrest mit 1 bis 6 Kohlenstoffatomen gebunden ist, steht; wobei jedes Kohlenstoffatom gegebenen- falls durch Halogen oder Hydroxy substituiert sein kann;
Ar für einen Homocyclus aus der Gruppe Phenyl, Naphthyl, Acenaphthyl, Tetrahydronaphthyl steht, wobei jeder Homocyclus gegebenenfalls durch 1, 2 oder 3 Substituenten substituiert ist, wobei jeder Substituent unabhängig aus der Gruppe Halogen, Halogenalkyl, Cyano, Alkyloxy und Morpholinyl ausgewählt ist;
Het für einen monocyclischen Heterocyclus aus der Gruppe *N*-Phenoxypiperidinyl, Piperidinyl, Furanyl, Thienyl, Pyridinyl und Pyrimidinyl oder einen bicyclischen Heterocyclus aus der Gruppe Benzothienyl, 2,3-Dihydrobenzo[1,4]- dioxinyl oder Benzo[1,3]dioxolyl steht; wobei jeder monocyclische und bicyclische Hetero- cyclus gegebenenfalls durch 1, 2 oder 3 Alkyl- oder Ar-Carbonyl-Substituenten substituiert sein kann; und
Halogen für einen Substituenten aus der Gruppe Fluor, Chlor und Brom steht.

3. Verwendung nach Anspruch 1 oder 2, wobei in der Formel (Ia) oder (Ib) R¹ für Wasserstoff, Halogen, Ar, Alkyl oder Alkyloxy steht.

4. Verwendung nach Anspruch 3, wobei R¹ für Halogen steht.

5. Verwendung nach einem der vorhergehenden Ansprüche, wobei in der Formel (Ia) oder (Ib) p gleich 1 ist.

6. Verwendung nach einem der vorhergehenden Ansprüche, wobei in der Formel (Ia) oder (Ib) R² für Wasserstoff, Alkyloxy oder Alkylthio steht.

7. Verwendung nach Anspruch 6, wobei R² für Alkyloxy steht.

8. Verwendung nach einem der vorhergehenden Ansprüche, wobei in der Formel (Ia) oder (Ib) R³ für Naphthyl, Phenyl oder Thienyl, die jeweils gegebenenfalls durch 1 oder 2 Substituenten aus der Gruppe Halogen und Halogenalkyl substituiert sind, steht.

9. Verwendung nach Anspruch 8, wobei R³ für Naphthyl steht.

10. Verwendung nach einem der vorhergehenden Ansprüche, wobei in der Formel (Ia) oder (Ib) q gleich 1 ist.

11. Verwendung nach einem der vorhergehenden Ansprüche, wobei in der Formel (Ia) oder (Ib) R⁴ und R⁵ jeweils unabhängig für Wasserstoff oder Alkyl stehen oder R⁴ und R⁵ gemeinsam und einschließlich des N, an das sie gebunden sind, einen Rest aus der Gruppe Imidazolyl, Triazolyl, Piperidinyl, Piperazinyl und Thiomorpholinyl bilden.

12. Verwendung nach Anspruch 11, wobei in der Formel (Ia) oder (Ib) R⁴ und R⁵ jeweils unabhängig für Wasserstoff oder Alkyl stehen.

13. Verwendung nach Anspruch 12, wobei R⁴ und R⁵ für C₁₋₄-Alkyl stehen.

14. Verwendung nach einem der vorhergehenden Ansprüche, wobei in der Formel (Ia) oder (Ib) R⁶ für Wasserstoff, Alkyl oder Halogen steht.

15. Verwendung nach Anspruch 14, wobei R⁶ für Wasserstoff steht.

16. Verwendung nach einem der vorhergehenden Ansprüche, wobei in der Formel (Ia) oder (Ib) r gleich 1 ist.

17. Verwendung nach einem der vorhergehenden Ansprüche, wobei in der Formel (Ia) oder (Ib) R⁷ für Wasserstoff steht.

18. Verwendung nach Anspruch 1, wobei in der Formel (Ia) oder (Ib) R¹ für Wasserstoff, Halogen, Ar, Alkyl oder Alkyloxy steht; p = 1; R² für Wasserstoff, Alkyloxy oder Alkylthio steht; R³ für Naphthyl, Phenyl oder Thienyl, die jeweils gegebenenfalls durch ein oder zwei Substituenten ausgewählt aus der Gruppe Halogen und Halogenalkyl substituiert sind, steht; q = 0, 1, 2 oder 3; R⁴ und R⁵ jeweils unabhängig für Wasserstoff oder Alkyl stehen oder R⁴ und R⁵ gemeinsam und einschließlich des N, an das sie gebunden sind, einen Rest aus der Gruppe Imidazolyl, Triazolyl, Piperidinyl, Piperazinyl und Thiomorpholinyl bilden; R⁶ für Wasserstoff, Alkyl oder Halogen steht; r gleich 1 ist und R⁷ für Wasserstoff steht.

19. Verwendung nach einem der vorhergehenden Ansprüche, wobei Alkyl für C₁-C₆-Alkyl steht.

20. Verwendung nach einem der vorhergehenden Ansprüche, wobei Halogenalkyl für Polyhalogen-C₁-C₆-alkyl steht.

21. Verwendung nach einem der vorhergehenden Ansprüche, wobei es sich bei der Verbindung um eine Verbindung der Formel (Ia) handelt.

22. Verwendung nach Anspruch 1, **dadurch gekennzeichnet, daß** die Verbindung aus der Gruppe bestehend aus:
o 1-(6-Brom-2-methoxychinolin-3-yl)-2-(3,5-difluorphenyl)-4-dimethylamino-1-phenylbutan-2-ol;
ο 1-(6-Brom-2-methoxychinolin-3-yl)-4-dimethylamino-2-naphthalin-1-yl-1-phenylbutan-2-ol;
o 1-(6-Brom-2-methoxychinolin-3-yl)-2-(2,5-difluorphenyl)-4-dimethylamino-1-phenylbutan-2-ol;
o 1-(6-Brom-2-methoxychinolin-3-yl)-2-(2,3-difluorphenyl)-4-dimethylamino-1-phenylbutan-2-ol;
o 1-(6-Brom-2-methoxychinolin-3-yl)-4-dimethylamino-2-(2-fluorphenyl)-1-phenylbutan-2-ol;
o 1-(6-Brom-2-methoxychinolin-3-yl)-4-dimethylamino-2-naphthalin-1-yl-1-p-tolylbutan-2-ol;
o 1-(6-Brom-2-methoxychinolin-3-yl)-4-methylamino-2-naphthalin-1-yl-1-phenylbutan-2-ol;
o 1-(6-Brom-2-methoxychinolin-3-yl)-4-dimethylamino-2-(3-fluorphenyl)-1-phenylbutan-2-ol und
o 1-(6-Brom-2-methoxychinolin-3-yl)-4-dimethylamino-2-phenyl-1-phenylbutan-2-ol;
einem pharmazeutisch annehmbaren Säure- oder Basenadditionssalz davon, einem *N*-Oxid davon oder einer stereochemisch isomeren Form davon ausgewählt ist.

23. Verwendung nach Anspruch 1, wobei die Verbindung aus der Gruppe bestehend aus:
1-(6-Brom-2-methoxychinolin-3-yl)-2-(2,3-difluorphenyl)-4-dimethylamino-1-phenylbutan-2-ol;
1-(6-Brom-2-methoxychinolin-3-yl)-4-dimethylamino-2-naphthalin-1-yl-1-phenylbutan-2-ol;
einem pharmazeutisch annehmbaren Säure- oder Basenadditionssalz davon, einem *N*-Oxid davon oder einer stereochemisch isomeren Form davon ausgewählt ist.

24. Verwendung nach Anspruch 1, wobei es sich bei der Verbindung um 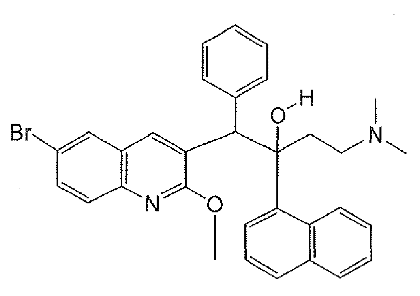 ein pharmazeutisch annehmbares Säure- oder Basenadditionssalz davon, ein *N*-Oxid davon oder eine stereochemisch isomere Form davon handelt.

25. Verwendung nach Anspruch 24, wobei es sich bei der Verbindung um 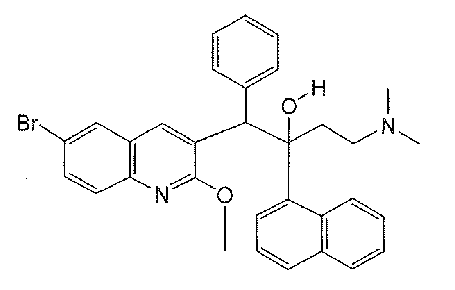 oder ein pharmazeutisch annehmbares Säureadditionssalz davon handelt.

26. Verwendung nach Anspruch 24, wobei es sich bei der
Verbindung um 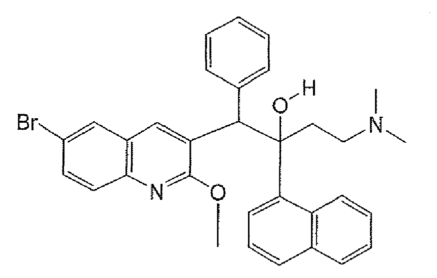 oder eine stereochemisch isomere Form davon handelt.

27. Verwendung nach Anspruch 24, wobei es sich bei der Verbindung um 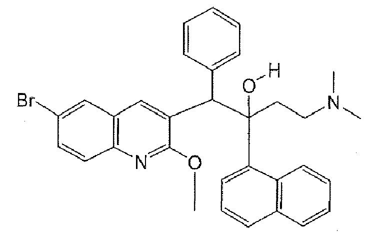 oder eine *N*-Oxidform davon handelt.

28. Verwendung nach Anspruch 24, wobei es sich bei der Verbindung um 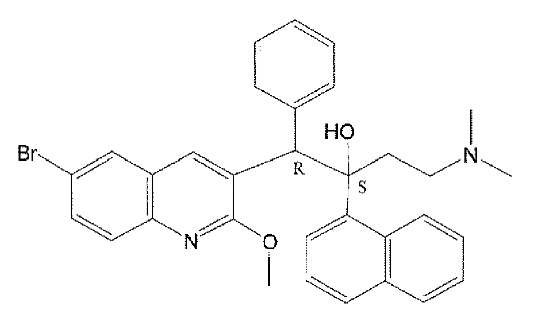 oder ein pharmazeutisch annehmbares Säureadditionssalz davon handelt.

29. Verwendung nach Anspruch 24, wobei es sich bei der Verbindung um 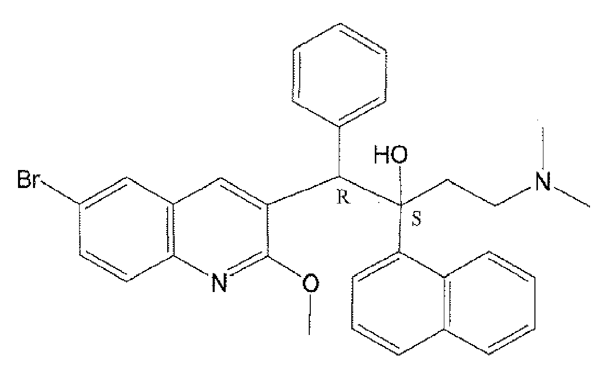 handelt.

## Revendications

1. Utilisation d'un composé de formule (Ia) ou (Ib) pour la fabrication d'un médicament destiné au traitement de la tuberculose latente, **caractérisée en ce que** le composé de formule (Ia) ou (Ib) est 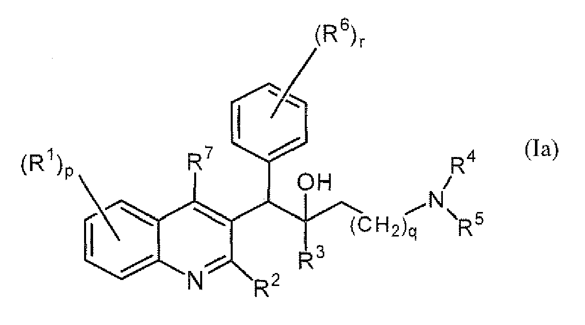 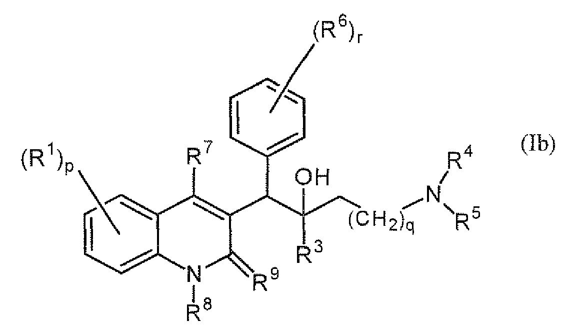 un sel d'addition d'acide ou de base pharmaceutiquement acceptable de celui-ci, une amine quaternaire de celui-ci, un *N*-oxyde de celui-ci, une forme tautomère de celui-ci ou une forme stéréochimiquement isomère de celui-ci, dans laquelle
R¹ est hydrogène, halogéno, halogénoalkyle, cyano, hydroxy, Ar, Het, alkyle, alkyloxy, alkylthio, alkyloxyalkyle, alkylthioalkyle, Ar- alkyle ou di (Ar) alkyle ;
p est un entier égal à 1, 2, 3 ou 4 ;
R² est hydrogène, hydroxy, mercapto, alkyloxy, alkyloxyalkyloxy, alkylthio, mono ou di(alkyl)amino ou un radical de formule 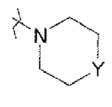 dans laquelle Y est CH₂,
R³ O, S, NH ou *N*-alkyle ; est alkyle, Ar, Ar-alkyle, Het ou Het- alkyle ;
q est un entier égal à zéro, 1, 2, 3 ou 4 ;
R⁴ et R⁵ chacun indépendamment sont hydrogène, alkyle ou benzyle ; ou
R⁴ et R⁵ ensemble et y compris le N auquel ils sont liés peuvent former un radical choisi dans le groupe de pyrrolidinyle, 2-pyrrolinyle, 3-pyrrolinyle, pyrrolyle, imidazolidinyle, pyrazolidinyle, 2-imidazolinyle, 2- pyrazolinyle, imidazolyle, pyrazolyle, triazolyle, pipéridinyle, pyridinyle, pipérazinyle, pyridazinyle, pyrimidinyle, pyrazinyle, triazinyle, morpholinyle et thiomorpholinyle, chacun desdits systèmes de cycle éventuellement substitué par alkyle, halogéno, halogénoalkyle, hydroxy, alkyloxy, amino, mono- ou dialkylamino, alkylthio, alkyloxyalkyle, alkylthioalkyle et pyrimidinyle ;
R⁶ est hydrogène, halogéno, halogénoalkyle, hydroxy, Ar, alkyle, alkyloxy, alkylthio, alkyloxyalkyle, alkylthioalkyle, Ar-alkyle o u di(Ar)alkyle ; ou
deux radicaux R⁶ vicinaux peuvent être pris ensemble pour former un radical bivalent de formule -CH=CH-CH=CH- ;
r est un entier égal à 1, 2, 3, 4 ou 5 ;
R⁷ est hydrogène, alkyle, Ar ou Het ;
R⁸ est hydrogène ou alkyle ;
R⁹ est oxo ; ou
R⁸ et R⁹ forment ensemble le radical =N-CH=CH- ;
alkyle est un radical hydrocarboné saturé droit ou ramifié ayant de 1 à 6 atomes de carbone ; ou un radical hydrocarboné saturé cyclique ayant de 3 à 6 atomes de carbone ; ou un radical hydrocarboné saturé cyclique ayant de 3 à 6 atomes de carbone lié à un radical hydrocarboné saturé droit ou ramifié ayant de 1 à 6 atomes de carbone ; chaque atome de carbone pouvant être éventuellement substitué par halogéno, hydroxy, alkyloxy ou oxo ;
Ar est un homocycle choisi dans le groupe de phényle, naphtyle, acénaphtyle, tétrahydronaphtyle, chaque homocycle éventuellement substitué par 1, 2 ou 3 substituants, chaque substituant choisi indépendamment dans le groupe d'hydroxy, halogéno, cyano, nitro, amino, mono- ou dialkylamino, alkyle, halogénoalkyle, alkyloxy, halogénoalkyloxy, carboxyle, alkyloxycarbonyle, aminocarbonyle, morpholinyle et mono- ou dialkylaminocarbonyle ;
Het est un hétérocycle monocyclique choisi dans le groupe de *N*-phénoxypipéridinyle, pipéridinyle, pyrrolyle, pyrazolyle, imidazolyle, furanyle, thiényle, oxazolyle, isoxazolyle, thiazolyle, isothiazolyle, pyridinyle, pyrimidinyle, pyrazinyle et pyridazinyle ; ou un hétérocycle bicyclique choisi dans le groupe de quinoléinyle, quinoxalinyle, indolyle, benzimidazolyle, benzoxazolyle, benzisoxazolyle, benzothiazolyle, benzisothiazolyle, benzofuranyle, benzothiényle,2,3- dihydrobenzo[1,4]dioxinyle ou benzo[1,3]dioxolyle ; chaque hétérocycle monocyclique et bicyclique peut éventuellement être substitué par 1, 2 ou 3 substituants choisis dans le groupe d'halogéno, hydroxy, alkyle, alkyloxy ou Ar- carbonyle ;
halogéno est un substituant choisi dans le groupe de fluoro, chloro, bromo et iodo ; et
halogénoalkyle est un radical hydrocarboné saturé droit ou ramifié ayant de 1 à 6 atomes de carbone ou un radical hydrocarboné saturé cyclique ayant de 3 à 6 atomes de carbone, un ou plusieurs atomes de carbone étant substitués par un ou plusieurs atomes halogéno.

2. Utilisation selon la revendication 1, **caractérisée en ce que**
R¹ est hydrogène, halogéno, cyano, Ar, Het, alkyle et alkyloxy ;
p est un entier égal à 1 ou 2 ;
R² est hydrogène, hydroxy, alkyloxy, alkyloxyalkyloxy, alkylthio ou un radical de formule 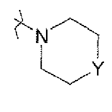 dans laquelle
R³ Y est O ; est alkyle, Ar, Ar-alkyle ou Het ;
q est un entier égal à zéro, 1, 2 ou 3 ;
R⁴ et R⁵ chacun indépendamment sont hydrogène, alkyle ou benzyle ; ou
R⁴ et R⁵ ensemble et y compris le N auquel ils sont liés peuvent former un radical choisi dans le groupe de pyrrolidinyle, imidazolyle, triazolyle, pipéridinyle, pipérazinyle, pyrazinyle, morpholinyle et thiomorpholinyle, chaque système de cycle éventuellement substitué par alkyle ou pyrimidinyle ;
R⁶ est hydrogène, halogéno ou alkyle ; ou
deux radicaux R⁶ vicinaux peuvent être pris ensemble pour former un radical bivalent de formule -CH=CH-CH=CH- ;
r est un entier égal à 1 ;
R⁷ est hydrogène ;
R⁸ est hydrogène ou alkyle ;
R⁹ est oxo ; ou
R⁸ et R⁹ forment ensemble le radical =N-CH=CH- ;
alkyle est un radical hydrocarboné saturé droit ou ramifié ayant de 1 à 6 atomes de carbone ; ou un radical hydrocarboné saturé cyclique ayant de 3 à 6 atomes de carbone ; ou un radical hydrocarboné saturé cyclique ayant de 3 à 6 atomes de carbone lié à un radical hydrocarboné saturé droit ou ramifié ayant de 1 à 6 atomes de carbone ; chaque atome de carbone pouvant être éventuellement substitué par halogéno ou hydroxy ;
Ar est un homocycle choisi dans le groupe de phényle, naphtyle, acénaphtyle, tétrahydronaphtyle, chaque homocycle éventuellement substitué par 1, 2 ou 3 substituants, chaque substituant choisi indépendamment dans le groupe d'halogéno, halogénoalkyle, cyano, alkyloxy et morpholinyle ;
Het est un hétérocycle monocyclique choisi dans le groupe de *N*-phénoxypipéridinyle, pipéridinyle, furanyle, thiényle, pyridinyle, pyrimidinyle ; ou un hétérocycle bicyclique choisi dans le groupe de benzothiényle, 2,3- dihydrobenzo[1,4]dioxinyle ou benzo[1,3]dioxolyle ; chaque hétérocycle monocyclique e t bicyclique peut éventuellement être substitué par 1, 2 ou 3 substituants alkyle ou Ar-carbonyle ; et halogéno est un substituant choisi dans le groupe de fluoro, chloro et bromo.

3. Utilisation selon la revendication 1 ou 2 , **caractérisée en ce que** dans la formule (Ia) ou (Ib), R¹ est hydrogène, halogéno, Ar, alkyle ou alkyloxy.

4. Utilisation selon la revendication 3, **caractérisée en ce que** R¹ est halogéno.

5. Utilisation selon l'une quelconque des revendications précédentes, **caractérisée en ce que** dans la formule (Ia) ou (Ib), p est égal à 1.

6. Utilisation selon l'une quelconque des revendications précédentes, **caractérisée en ce que** dans la formule (Ia) ou (Ib), R² est hydrogène, alkyloxy ou alkylthio.

7. Utilisation selon la revendication 6, **caractérisée en ce que** R² est alkyloxy.

8. Utilisation selon l'une quelconque des revendications précédentes, **caractérisée en ce que** dans la formule (Ia) ou (Ib), R³ est naphtyle, phényle ou thiényle, chacun éventuellement substitué par 1 ou 2 substituants choisis dans le groupe d'halogéno et halogénoalkyle.

9. Utilisation selon la revendication 8, **caractérisée en ce que** R³ est naphtyle.

10. Utilisation selon l'une quelconque des revendications précédentes, **caractérisée en ce que** dans la formule (Ia) ou (Ib), q est égal à 1.

11. Utilisation selon l'une quelconque des revendications précédentes, **caractérisée en ce que** dans la formule (Ia) ou (Ib), R⁴ et R⁵ sont chacun indépendamment hydrogène ou alkyle ou R⁴ et R⁵ ensemble et y compris le N auquel ils sont liés forment un radical choisi dans le groupe d'imidazolyle, triazolyle, pipéridinyle, pipérazinyle et thiomorpholinyle.

12. Utilisation selon la revendication 11, **caractérisée en ce que** dans la formule (Ia) ou (Ib), R⁴ et R⁵ chacun indépendamment sont hydrogène ou alkyle.

13. Utilisation selon la revendication 12, **caractérisée en ce que** R⁴ et R⁵ sont C₁₋₄alkyle.

14. Utilisation selon l'une quelconque des revendications précédentes, **caractérisée en ce que** dans la formule (Ia) ou (Ib), R⁶ est hydrogène, alkyle ou halogéno.

15. Utilisation selon la revendication 14, **caractérisée en ce que** R⁶ est hydrogène.

16. Utilisation selon l'une quelconque des revendications précédentes, **caractérisée en ce que** dans la formule (Ia) ou (Ib), r est égal à 1.

17. Utilisation selon l'une quelconque des revendications précédentes, **caractérisée en ce que** dans la formule (Ia) ou (Ib), R⁷ est hydrogène.

18. Utilisation selon la revendication 1, **caractérisée en ce que** dans la formule (Ia) ou (Ib), R¹ est hydrogène, halogéno, Ar, alkyle ou alkyloxy ; p = 1 ; R² est hydrogène, alkyloxy ou alkylthio ; R³ est naphtyle, phényle ou thiényle, chacun éventuellement substitué par 1 ou 2 substituants choisis dans le groupe d'halogéno et halogénoalkyle ; q = 0, 1, 2 ou 3 ; R⁴ et R⁵ chacun indépendamment sont hydrogène ou alkyle ou R⁴ et R⁵ ensemble et y compris le N auquel ils sont liés forment un radical choisi dans le groupe d'imidazolyle, triazolyle, pipéridinyle, pipérazinyle et thiomorpholinyle ; R⁶ est hydrogène, alkyle ou halogéno ; r est égal à 1 et R⁷ est hydrogène.

19. Utilisation selon l'une quelconque des revendications précédentes, **caractérisée en ce qu'**alkyle représente C₁₋₆alkyle.

20. Utilisation selon l'une quelconque des revendications précédentes, **caractérisée en ce qu'**halogénoalkyle représente polyhalogénoC₁₋₆alkyle.

21. Utilisation selon l'une quelconque des revendications précédentes, **caractérisée en ce que** le composé est un composé selon la formule (Ia).

22. Utilisation selon la revendication 1, **caractérisée en ce que** le composé est choisi dans le groupe constitué de :
o 1-(6-bromo-2-méthoxy-quinoléin-3-yl)-2-(3,5-difluoro-phényl)-4-diméthylamino-1-phényl-butan-2-ol ;
o 1-(6-bromo-2-méthoxy-quinoléin-3-yl)-4-diméthylamino-2-naphtalèn-1-yl-1-phényl-butan-2-ol ;
o 1-(6-bromo-2-méthoxy-quinoléin-3-yl)-2-(2,5-difluoro-phényl)-4-diméthylamino-1-phényl-butan-2-ol ;
o 1-(6-bromo-2-méthoxy-quinoléin-3-yl)-2-(2,3-difluoro-phényl)-4-diméthylamino-1-phényl-butan-2-ol ;
o 1-(6-bromo-2-méthoxy-quinoléin-3-yl)-4-diméthylamino-2-(2-fluoro-phényl)-1-phényl-butan-2-ol ;
o 1-(6-bromo-2-méthoxy-quinoléin-3-yl)-4-diméthylamino-2-naphtalèn-1-yl-1-p-tolyl-butan-2-ol ;
o 1-(6-bromo-2-méthoxy-quinoléin-3-yl)-4-méthylamino-2-naphtalèn-1-yl-1-phényl-butan-2-ol ;
o 1-(6-bromo-2-méthoxy-quinoléin-3-yl)-4-diméthylamino-2-(3-fluoro-phényl)-1-phényl-butan-2-ol ; et
o 1-(6-bromo-2-méthoxy-quinoléin-3-yl)-4-diméthylamino-2-phényl-1-phényl-butan-2-ol ;
un sel d'addition d'acide ou de base pharmaceutiquement acceptable de ceux-ci, un *N*-oxyde de ceux-ci ou une forme stéréochimiquement isomère de ceux-ci.

23. Utilisation selon la revendication 1, **caractérisée en ce que** le composé est choisi dans le groupe constitué de
o 1-(6-bromo-2-méthoxy-quinoléin-3-yl)-2-(2,3-difluoro-phényl)-4-diméthylamino-1-phényl-butan-2-ol ;
o 1-(6-bromo-2-méthoxy-quinoléin-3-yl)-4-diméthylamino-2-naphtalèn-1-yl-1-phényl-butan-2-ol ;
un sel d'addition d'acide ou de base pharmaceutiquement acceptable de ceux-ci, un *N*-oxyde de ceux-ci ou une forme stéréochimiquement isomère de ceux-ci.

24. Utilisation selon la revendication 1, **caractérisée en ce que** le composé est 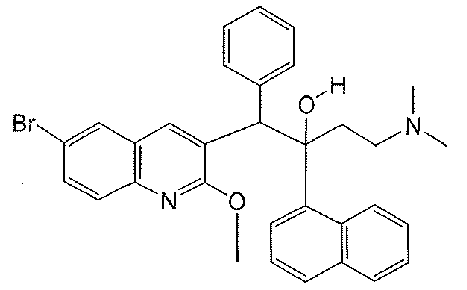 un sel d'addition d'acide ou de base pharmaceutiquement acceptable de celui-ci, un *N*-oxyde de celui-ci ou une forme stéréochimiquement isomère de celui-ci.

25. Utilisation selon la revendication 24, **caractérisée en ce que** le composé est 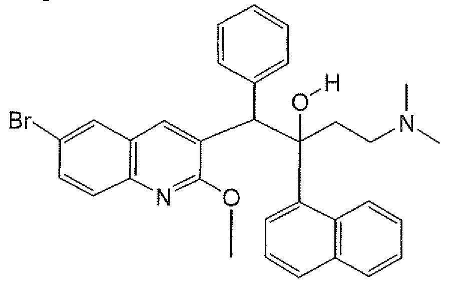 ou un sel d'addition d'acide pharmaceutiquement acceptable de celui-ci.

26. Utilisation selon la revendication 24, **caractérisée en ce que** le composé est 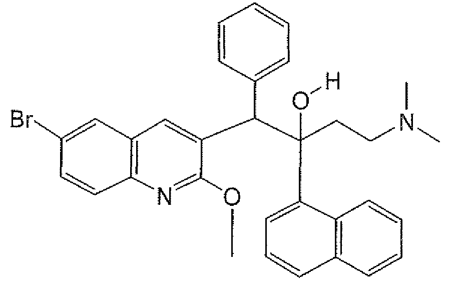 ou une forme stéréochimiquement isomère de celui-ci.

27. Utilisation selon la revendication 24, **caractérisée en ce que** le composé est 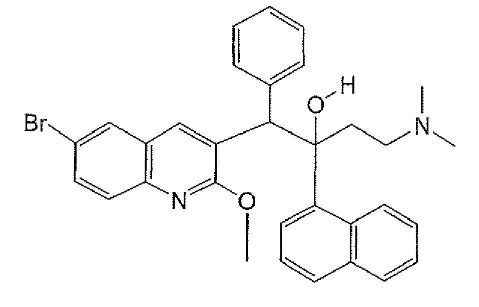 ou une forme *N*-oxyde de celui-ci.

28. Utilisation selon la revendication 24, **caractérisée en ce que** le composé est 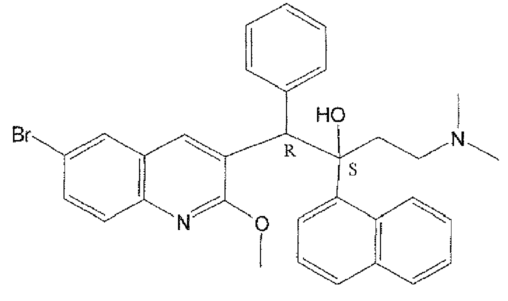 ou un sel d'addition d'acide pharmaceutiquement acceptable de celui-ci.

29. Utilisation selon la revendication 28, **caractérisée en ce que** le composé est
